(19) Europäisches Patentamt
European Patent Office
Office européen des brevets

(11) **EP 3 702 474 A1**

(12) **EUROPEAN PATENT APPLICATION**

(43) Date of publication:
**02.09.2020 Bulletin 2020/36**

(51) Int Cl.:
*C12Q 1/6869* (2018.01)     *G16B 30/20* (2019.01)

(21) Application number: **19159434.0**

(22) Date of filing: **26.02.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **QIAGEN GmbH**
**40724 Hilden (DE)**

(72) Inventors:
- **Hanselmann, Thomas**
  **8708 Männedorf (CH)**
- **Lutz, Raimund**
  **6330 Cham (CH)**

- **Unterer, Thomas**
  **8853 Lachen (CH)**
- **Hassler, Kai**
  **6005 Luzern (CH)**
- **Quintel, Harald**
  **8266 Steckborn (CH)**
- **Lutze, Konstantin**
  **8713 Uerikon (CH)**
- **Schuster, Guido**
  **8712 Stäfa (CH)**

(74) Representative: **Roth, Carla et al**
**König-Szynka-Tilmann-von Renesse**
**Patentanwälte Partnerschaft mbB**
**Mönchenwerther Straße 11**
**40545 Düsseldorf (DE)**

(54) **SEQUENCING METHOD AND KIT**

(57)     The present invention provides advantageous sequencing methods, systems and kits that are suitable in the field of next generation sequencing, wherein a mixture of nucleotide analogues is used, wherein in the same category of nucleotide analogues a mixture of molecules is used that are labelled with different detectable labels.

EP 3 702 474 A1

**Description**

**FIELD OF THE INVENTION**

The present invention belongs to the field of sequencing, in particular next-generation-sequencing, where a nucleic acid template such as single-stranded DNA or RNA are sequenced by synthesis in single steps, using nucleotide analogues comprising a detectable label. Also provided are kits and systems suitable for sequencing a nucleic acid template.

**BACKGROUND OF THE INVENTION**

Nucleic acid sequencing is the process of determining the precise order of nucleotides within a nucleic acid molecule. In DNA sequencing, it includes the step of determining the order of the four nucleobases-adenine, guanine, cytosine, and thymine-in a strand of DNA. Traditional sequencing methods (e.g. Sanger sequencing) are being replaced by next-generation sequencing technologies. Next-generation sequencing (NGS) allows to determine the sequence of DNA and RNA (which usually is reverse transcribed first to cDNA) molecules in a massively parallel manner. Next-generation sequencing is widely used to sequence e.g. exomes, genomes, circulating nucleic acids, targeted panels or mitochondrial DNA.

Prior art next-generation sequencing approaches are reviewed e.g. in Goodwin et al., Nature Reviews, June 2016, Vol. 17: pp. 333 - 351 "Coming of age: ten years of next-generation sequencing technologies", Yohe et al., Arch Pathol Lab Med, November 2017, Vol. 141: pp. 1544 - 1557 "Review of Clinical Next-Generation Sequencing" and Masoudi-Nejad, Chapter 2 "Emergence of Next-Generation Sequencing in "Next Generation Sequencing and Sequence Assembly" SpringerBriefs in Systems Biology, 2013, all herein incorporated by reference. Current next-generation sequencing approaches share common sample preparation steps to provide the nucleic acid template that is then sequenced. These common steps usually include steps such as nucleic acid extraction, library preparation, target enrichment and clonal amplification. The so prepared nucleic acid template is then ready to be sequenced. A widely used sequencing approach is referred to as sequencing by synthesis (SBS). In most SBS approaches, DNA is clonally amplified on a solid surface. Having multiple identical copies of a DNA fragment in a defined area ensures that the signal used to determine the sequence can be distinguished from background noise.

Sequencing by synthesis (SBS) is a term used to describe numerous DNA-polymerase-dependent methods. In sequencing by synthesis (SBS) approaches, a polymerase is used and a signal, such as a fluorophore or a change in ionic concentration, identifies the incorporation of a nucleotide into an elongating strand. Commonly used SBS approaches can be classified e.g. as cyclic reversible termination (CRT) or as single-nucleotide addition (SNA). CRT approaches are widely used in the art and use a nucleotide analogue comprising a detectable label, e.g. a color-label such as a fluorescent label. Different types of nucleotide analogous comprising a detectable label can be used for CRT based methods. Labelled nucleotide analogues commonly used in CRT based methods are also referred to sometimes as reversible terminators or terminator molecules. Different embodiments are used, such as e.g. 3'-O-blocked reversible terminators and 3'-unblocked reversible terminators (see e.g. Chen et al, 2013 "The History and Advances of Reversible Terminators Used in New Generations of Sequencing Technology" Genomics Proteomics Bioinformatics 11 (2013) 34-40 and Chen et al, 2014, "DNA polymerases drive DNA sequencing-by synthesis technologies: both past and present", Frontiers in Microbiology, Volume 5, Article 305, p. 1 to 11; herein incorporated by reference). The 3'O-blocked reversible terminators are similar to those used in Sanger sequencing, in which the ribose 3'-OH group of the nucleotide is blocked by a terminating group, also referred to as capping moiety, thus preventing elongation after the incorporation of a labelled nucleotide analogue. However, the 3'O-blocked reversible terminators used in next generation sequencing methods comprise a reversible terminating group which accordingly can be removed and the label, e.g. a fluorescent dye, is also attached removable, e.g. via a cleavable linker. The main steps of commonly used sequencing approaches are summarized subsequently. To begin the sequencing process, an immobilized DNA template is usually primed by a sequence that is complementary to an adapter region (or a self-priming DNA template is used), which will initiate polymerase binding to this double-stranded DNA (dsDNA) region. During each sequencing cycle, a mixture of labelled nucleotide analogues, e.g. four individually labelled and 3'-blocked deoxynucleotides (dNTPs), is added. The incorporation of a single labelled nucleotide analogue to each elongating complementary strand provides a hybrid which comprises the detectable label of the nucleotide analogue that was incorporated. Unbound labelled nucleotide analogues are removed and the immobilized DNA template is imaged to identify based on the detectable label which nucleotide was incorporated into the hybrid thereby allowing to identify the complementary base of the nucleic acid template. The detectable label (e.g. fluorophore) and the capping moiety are then removed and a new sequencing cycle can begin to identify the next consecutive base.

Commercially available platforms using sequencing by synthesis based on cyclic reversible termination (CRT) are MiSeq, HiSeq, NextSeq etc. (Illumina) and GeneReader (QIAGEN) (see also Goodwin et al, 2016). In the Illumina platforms, after solid-phase template enrichment, a mixture of primers, DNA polymerase and nucleotide analogues are added to the flow cell. Each nucleotide analogue is blocked by a 3'-O-azidomethyl group and is labelled with a base-specific, cleavable fluorophore. During each sequencing cycle, the nucleic acid templates in each cluster will incorporate just one nucleotide analogue as the blocked 3' group prevents additional incorporations. After base incorporation, unincorporated nucleotide analogues are washed away and the slide is imaged by total internal reflection fluorescence (TIRF) microscopy using either two or four laser channels. The colour (or the lack or mixing of colours in the two-channel system used by NextSeq) identifies which base was incorporated in each cluster. The fluorescent dye is then cleaved and the 3'-OH is regenerated with a reducing agent (e.g. tris(2-carboxyethyl)phosphine ((TCEP)). The sequencing cycle of nucleotide analogue addition, elongation and cleavage is then repeated to identify the next base. In the GeneReader platform, after bead-based template enrichment, a mixture of primers, DNA polymerase and modified nucleotides are added to the flow cell. Each nucleotide analogue is blocked by a 3'-O-allyl group and some of the bases are labelled with a base-specific, cleavable fluorophore. After base incorporation, unincorporated nucleotide analogues are washed away and the slide can be imaged with a fluorescence microscope using four laser channels or LEDs. The dye is then cleaved and the 3'-OH is regenerated with the reducing agent (e.g. mixture of palladium and $P(PhSO_3Na)_3$ (TPPTS)).

However, the prior art methods have drawbacks. The sequencing systems that use a four-color-system, wherein each nucleotide analogue is labelled with a different fluorescent dye, while being robust, have the drawback that four images need to be taken per sequencing cycle which is time consuming and thus leads to longer turn-around times. The known systems of Illumina (NextSeq) which use a two-color system, wherein a nucleotide analogue is labelled with two fluorescent dyes (i.e. two fluorescent dyes linked to a single nucleotide molecule), while allowing a higher throughput because less images are required, *inter alia* have the drawback that they are not as robust as the known four-color systems.

The invention aims at avoiding drawbacks of the prior art methods. It would be advantageous to provide sequencing methods and systems that combine the advantages of a good throughput and robustness. It is therefore one object to provide an improved method for determining the sequence of a nucleic acid template. It is another object of the invention to provide a method for determining the sequence of a nucleic acid template which increases the throughput while maintaining the robustness of a four-color system. It is furthermore an objective to find efficient ways to decode the sequence of the nucleic acid template by increasing total system throughput, i.e. number of total decoded bases per time, subject to low error rates.

## SUMMARY OF THE INVENTION

The present invention belongs to the field of sequencing, in particular next generation sequencing. As in prior art methods, the present technology uses four different nucleotide analogue types, wherein each type is representative of a nucleobase that is comprised in nucleic acid molecules (the nucleobases being A, C, G, T and U). Hence, each nucleotide analogue type specifically binds to a unique DNA or RNA nucleotide in the sequencing process. The present invention uses an advantageous labelling strategy for the four nucleotide analogue types which is based on the use of three different detectable labels, preferably three fluorescent dyes of different colours, wherein different detectable labels are mixed within a nucleotide analogue type. The present invention thereby allows to use a coding scheme, wherein the nucleotide types are encoded by 4 codewords that are generated by the three different detectable labels. As is described herein, the 4 codewords advantageously may have a Hamming distance of 2. The present technology achieves important advantages compared to prior art methods. Compared to the prior art two-color system that uses nucleotide analogues wherein nucleotide analogue molecules are labelled with two dyes (dual labelled nucleotides), the present technology has the advantage in being more robust and therefore having a lower error-rate. Furthermore, single-labelled nucleotide analogues can be used. Single-linked labels are smaller in size than dual-linked labels and thus interfere less with the polymerase-based synthesis step which is a further advantage compared to the prior art system. As is demonstrated in the examples, the present technology is furthermore as robust as the prior art four-color based labelling system and therefore has a low error rate, while the throughput can be considerably increased because the present technology is based on the use of three instead of four different detectable labels. Moreover, considerably less complicated detection devices can be used in conjunction with the present invention. E.g. a detection system can be used which is based on a dichroic filter. A dichroic filter can typically be applied for up to three different detectable labels and thus can be advantageously utilized for the three-detectable label based system of the present invention. This is not possible with various state of the art four-detectable label based systems when using standard commercially available dyes, whose excitation and emission spectra are too close to each other to accommodate a fourth dye. Such systems therefore often need to utilize four switchable filter cubes instead of dichroic filters. Hence, a technology is provided for determining the sequence of a nucleic acid template which achieves high decoding robustness and acquisition of at least the same information content as a four-colour based sequencing method, while increasing the throughput. As throughput is proportional to e.g. the number of images taken to detect three different fluorescent labels (colors), the proposed invention improves throughput directly by 1/4 but having the same low error rate of a four-color-system. Also provided is a model to show the robustness of the decoding from the use of a three detectable label based system, which preferably is a three-color-system, which shows equivalence to the current four-color-system but having to take only three instead of four images per sequencing step and superiority to two-colour systems in terms of decoding robustness. Further advantages and advantageous aspects and embodiments are described below.

According to a first aspect, a method for determining the sequence of a nucleic acid template is provided, said method comprising

a) contacting the nucleic acid template in a sequencing reaction with

(i) a nucleotide analogue of a first type, e.g. comprising the nucleobase A, wherein at least a portion or all of the nucleotide analogue molecules of the first type comprise a first detectable label;
(ii) a nucleotide analogue of a second type, e.g. comprising the nucleobase T or U, wherein at least a portion or all of the nucleotide analogue molecules of the second type comprise a second detectable label;
(iii) a nucleotide analogue of a third type, e.g. comprising the nucleobase G, wherein at least a portion or all of the nucleotide analogue molecules of the third type comprise a third detectable label;
(iv) a nucleotide analogue of a fourth type, e.g. comprising the nucleobase C, wherein the nucleotide analogue of the fourth type is provided by

(aa) nucleotide analogue molecules of the fourth type comprising the first detectable label,
(bb) nucleotide analogue molecules of the fourth type comprising the second detectable label, and
(cc) nucleotide analogue molecules of the fourth type comprising the third detectable label;
and

b) identifying the nucleotide analogue type incorporated in the sequencing reaction based on the detectable label(s) of the incorporated nucleotide analogue type thereby determining the sequence of the nucleic acid template.

According to a second aspect, a method for determining the sequence of a nucleic acid template is provided, said method comprising

a) contacting the nucleic acid template in a sequencing reaction with

(i) a nucleotide analogue of a first type, e.g. comprising the nucleobase A, wherein the nucleotide analogue of the first type comprises (aa) nucleotide analogues molecules of the first type comprising a first detectable label, and (bb) nucleotide analogue molecules of the first type comprising a second detectable label;
(ii) a nucleotide analogue of a second type, e.g. comprising the nucleobase T or U, wherein the nucleotide analogue of the second type comprises (aa) nucleotide analogue molecules of the second type comprising the first detectable label, and (bb) nucleotide analogue molecules of the second type comprising a third detectable label;
(iii) a nucleotide analogue of a third type, e.g. comprising the nucleobase G, wherein the nucleotide analogue of the third type comprises (aa) nucleotide analogue molecules of the third type comprising the second detectable label, and (bb) nucleotide analogue molecules of the third type comprising the third detectable label;
(iv) a nucleotide analogue of a fourth type, e.g. comprising the nucleobase C, wherein the nucleotide analogue of the fourth type does not comprise a detectable label; and

b) identifying the nucleotide analogue type incorporated in the sequencing reaction, wherein the incorporation of the first, second and third type of nucleotide analogue is determined based on the detectable labels of the incorporated type of nucleotide analogue and the incorporation of the fourth type is determined based on the absence of a detectable label.

According to a third aspect, a method for sequencing a nucleic acid template comprising four different nucleobases is provided, said method comprising contacting the nucleic acid template in a sequencing reaction with four different nucleotide analogue types that are complementary to the four different nucleobases of the nucleic acid template, wherein the method comprises detecting three different detectable labels that are comprised in the four different nucleotide analogue types, wherein the four different nucleobases are encoded with four 3-bit codewords generated by signals provided by the three different detectable labels that are comprised in the four different nucleotide analogue types and wherein the four 3-bit codewords have a Hamming distance of 2.

According to a fourth aspect, a kit is provided for determining the sequence of a nucleic acid template, wherein the kit comprises
per variant A

(i) a nucleotide analogue of a first type, e.g. comprising the nucleobase A, wherein at least a portion or all of the nucleotide analogue molecules of the first type comprise a first detectable label;

(ii) a nucleotide analogue of a second type, e.g. comprising the nucleobase T or U, wherein at least a portion or all of the nucleotide analogue molecules of the second type comprise a second detectable label;

(iii) a nucleotide analogue of a third type, e.g. comprising the nucleobase G, wherein at least a portion or all of the nucleotide analogue molecules of the third type comprise a third detectable label;

(iv) a nucleotide analogue of a fourth type, e.g. comprising the nucleobase C, wherein the nucleotide analogue of the fourth type comprises

(aa) nucleotide analogue molecules of the fourth type comprising the first detectable label,

(bb) nucleotide analogue molecules of the fourth type comprising the second detectable label, and

(cc) nucleotide analogue molecules of the fourth type comprising the third detectable label; or

per variant B

(i) a nucleotide analogue of a first type, e.g. comprising the nucleobase A, wherein the nucleotide analogue of the first type comprises (aa) nucleotide analogues molecules of the first type comprising a first detectable label, and (bb) nucleotide analogue molecules of the first type comprising a second detectable label;

(ii) a nucleotide analogue of a second type, e.g. comprising the nucleobase T or U, wherein the nucleotide analogue of the second type comprises (aa) nucleotide analogue molecules of the second type comprising the first detectable label, and (bb) nucleotide analogue molecules of the second type comprising a third detectable label;

(iii) a nucleotide analogue of a third type, e.g. comprising the nucleobase G, wherein the nucleotide analogue of the third type comprises (aa) nucleotide analogue molecules of the third type comprising the second detectable label, and (bb) nucleotide analogue molecules of the third type comprising the third detectable label;

(iv) a nucleotide analogue of a fourth type, e.g. comprising the nucleobase C, wherein the nucleotide analogue of the fourth type does not comprise a detectable label.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only. Various changes and modifications within the spirit and scope of the disclosed invention will become readily apparent to those skilled in the art from reading the following.

## BRIEF DESCRIPTION OF THE FIGURES

**Fig. 1:** Comparison of the three-colour system of the present invention according to different embodiments with a prior art two-colour system.

**Fig. 2:** Demonstrates that the three-colour system of the present invention results in a Hamming distance of 2.

**Fig. 3:** Demonstrates that lead and lag operation can be compensated by the present invention.

## DETAILED DESCRIPTION OF THE INVENTION

A nucleic acid template usually comprises 4 different bases which are in the sequencing technology of the present invention encoded with 4 codewords that are generated based on three different detectable labels. The four nucleotide analogues types are distinguishable based on the used detectable label(s). Accordingly, the first, second and third detectable label used for labelling the four different nucleotide analogues are distinguishable from each other. The first, second and third detectable label may be provided by optically detectable labels, preferably by different fluorescent dyes. Each fluorescent dye which provides a different detectable label may have a different excitation/emission spectra thereby allowing to distinguish between the different detectable labels.

As disclosed herein, the labelling strategy of the present invention has the advantage that the 4 codewords may have a Hamming distance of 2, while the effort required to determine the codewords based on the detectable labels can be reduced compared to prior art methods. The three detectable label based sequencing technology disclosed herein uses a coding scheme, wherein the error rates are not increased compared to the well-established, four-color based systems, while at the same time allowing to reduce the number of detection signals to be taken per synthesis cycle. This is explained in the following for the sake of simplicity predominantly referring to the preferred embodiment wherein three optically detectable labels, such as three different fluorescent dyes, are used as detectable labels. The same considerations apply when using other distinguishable detectable labels.

The three detectable label based sequencing technology of the present disclosure allows for a color-coding with only three fluorescent dyes, while having the same information content as a four-color based system, that uses one color (fluorescent label) for each nucleotide analogue type, as it is currently common practice. The present method is thereby as good as prior art methods. Specifically, the present technology allows to identify the four nucleotides of a nucleic acid template in a sequencing reaction using four nucleotide analogue types that are in sum marked with three different single detectable labels, such as three fluorescent dyes of different colors. In contrast to prior art methods wherein all nucleotide analogue molecules of the same type are labelled with the same fluorescent dye, the invention uses at least for one type of nucleotide analogue a mixture of nucleotide analogue molecules, wherein in said mixture nucleotide analogue molecules of the same type are labelled with different fluorescent labels. As is described in further detail below, it is advantageous that each nucleotide analogue molecule that comprises a detectable label only comprises a single detectable label, such as a single fluorescent dye, and not two detectable labels such as e.g. two fluorescent dyes (as it is known from prior art systems, such as dual dyes on one nucleotide analogue molecule).

The present invention allows the statistical mixing of differently (single) labelled nucleotide analogue molecules of the same type, thereby obtaining mixed color coded signals for the same nucleotide analogue type in the sequencing reaction, similarly as it can be achieved with a nucleotide analogue type that is labelled with dual-dyes, i.e. wherein a single nucleotide molecule is labelled with multiple dyes simultaneously. One important advantage of using single-labelled nucleotide analogue molecules in comparison to prior art methods wherein nucleotide analogue molecules comprising two fluorescent dyes are used, is that a nucleotide analogue molecule that is labelled with a single detectable label, such as a single fluorescent dye, is much smaller in size and thus does less interfere with the polymerase-based synthesis step. Furthermore, the three detectable label based coding that is used according to the present invention is better in terms of error detection (as codewords have a Hamming distance of 2) and thus is less error prone compared to a prior art method that uses a two-color based labelling system using dual labelled dyes, which have only a Hamming distance of 1 and thus cannot detect decoding errors.

The statistical mixing of single-labels, such as a fluorescent dye, may be formulated mathematically by a binary code vector where each of the digits indicates if a dye is present or not and the codeword describes a nucleotide category to be detected. As there are regularly only four nucleotides to be identified in the sequencing process, four codewords are sufficient to encode these. As the present invention uses three detectable labels instead of the minimum of two detectable labels, this kind of encoding enables redundancy which can be used to make decoding more robust with regards to degrading signal to noise ratio as well as for the effect of phasing and pre-phasing which are side-effects of an imperfect synthesis step. Advantageously, the principle of the present invention allows to achieve a Hamming distance of 2, while only using three different detectable labels, such as three different color-labels. Comparison of the 3-detectable label based system of the present invention with the 2-detectable label based prior art systems reveals distinct advantages of the 3-detectable label based system. While in a 2-detectable label system no-, single- and dual-detectable label linked to nucleotides represent the binary code, resulting in a Hamming distance of 1, the 3-detectable label system allows to achieve a Hamming distance of 2 by the following code choice: [001], [010], [100], [111] (see method according to the first aspect) or, in another embodiment, by [011], [101], [110], [000] (see method according to the second aspect). This coding and labelling strategy, respectively, circumvents the need for dual-detectable labels.

As is furthermore described herein, the single labelled nucleotide analogue molecules of the same type may also be provided in form of a composition that additionally comprises nucleotide analogue molecules of the same type that do not comprise a detectable label. As is discussed in the background section, it is possible to use a mixture of labelled and non-labelled nucleotide analogue molecules of the same type in a next generation sequencing method (see e.g. the GeneReader system). This is also possible in conjunction with the advantageous three detectable label based sequencing system of the present invention.

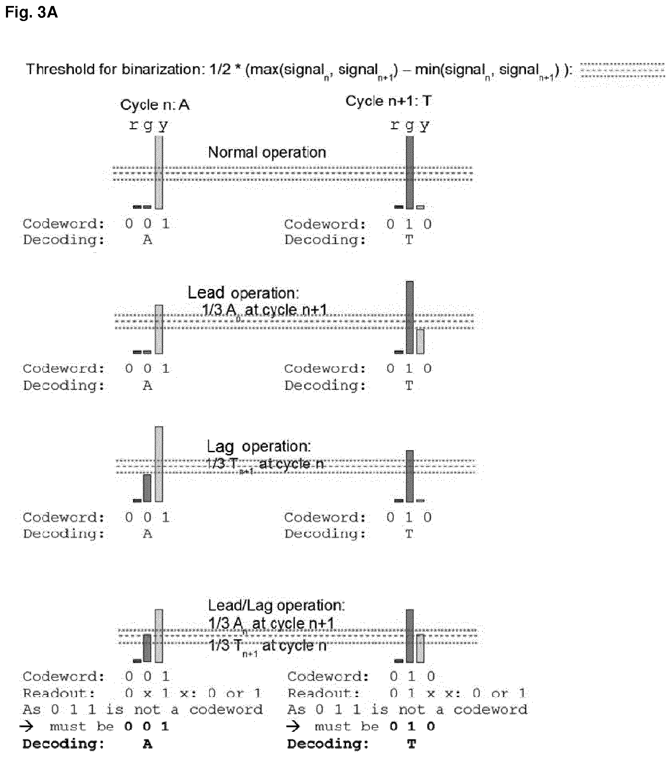

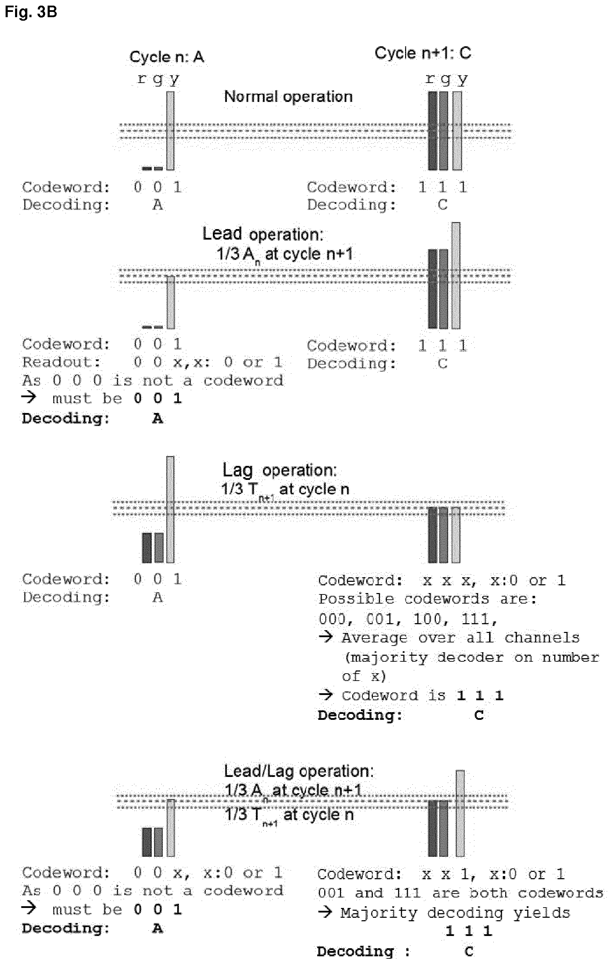

A core aspect of the present invention is the three detectable label based coding and decoding scheme which allows for higher system throughput e.g. by reducing the number of images to be taken per sequencing step to identify the different fluorescent dyes that were incorporated in the sequencing reaction. As is demonstrated herein, when going from a four-color to a three-color binary system there is equivalent information contained. Other coding/decoding schemes allow trade-off between number of images taken and error correction capability, where with specific optimization the best solution with respect to the desired target functionality can be achieved in the sequencing method. The present technology allows the selection of four codewords encoding the four nucleotides of the nucleic acid template to be deciphered from all the possible distinguishable codewords. In a binary three-detectable label based system, such as a three-color system, there are eight possible codewords where two sets exist, with four codewords each, such that all codewords have a pairwise Hamming distance of 2. A Hamming distance of 2 enables the detection of potential signal errors when decoding and even correcting for certain errors, in particular when using the strength of the raw detection signal (see e.g. Figure 3A and 3B), inclusively correcting phasing and pre-phasing errors that lead to signal degradation at higher cycle numbers. Furthermore, extensions of the binary decoding scheme to multi-level decoding schemes are shown, allowing to reduce the number of images per sequencing cycle down to two but still having redundancy for better decoding compared to current state-of-the-art systems. Moreover, as explained above the three detectable label based system of the present invention can advantageously utilize a dichroic filter in the detection device instead of filter cubes which are typically applied in conjunction with commercially used dyes. Dichroic filters can typically be applied to filter not more than three different optical signals, as optical signals (e.g. emission spectra) are too close together to accommodate a fourth dye. Thus, dichroic filters are not suitable for various the state of the art four-detectable label based systems. As the present invention is based on a labelling system comprising three different detectable labels, dichroic filters can be applied for detection. This has the advantage of being cheaper and less complicated. Another advantage of the dichroic filter in combination with the present method is a higher achievable throughout, as no mechanical adjustment is required (in comparison to commonly applied devices necessitating switching of filter cubes).

E.g. a dichroic filter can be made for the three colour combination Alexa488-N3-dTTP; Cy5-N3-dTTP; ROX-N3-dTTP.

The present invention is now explained in further detail with respect to the different aspects.

**The method according to the first aspect**

According to one aspect, the core concept of the present invention as explained above is implemented by providing one type of nucleotide analogue (e.g. comprising the nucleobase C) as mixture of differently labelled nucleotide analogue molecules. Within this nucleotide analogue type (e.g. comprising the nucleobase C), the nucleotide analogue molecules are labelled with the three detectable labels that are used for determining the sequence. Hence, there are three different kinds of labelled nucleotide analogue molecules of this type (e.g. comprising the nucleobase C): one kind comprising the first label, the second kind comprising the second label and the third type comprising the third label. As disclosed herein, there may be additionally non-labelled nucleotide molecules of this type.

Hence, according to a first aspect, a method for determining the sequence of a nucleic acid template is provided, said method comprising

a) contacting the nucleic acid template in a sequencing reaction with

(i) a nucleotide analogue of a first type, e.g. comprising the nucleobase A, wherein at least a portion or all of the nucleotide analogue molecules of the first type comprise a first detectable label;
(ii) a nucleotide analogue of a second type, e.g. comprising the nucleobase T or U, wherein at least a portion or all of the nucleotide analogue molecules of the second type comprise a second detectable label;
(iii) a nucleotide analogue of a third type, e.g. comprising the nucleobase G, wherein at least a portion or all of the nucleotide analogue molecules of the third type comprise a third detectable label;
(iv) a nucleotide analogue of a fourth type, e.g. comprising the nucleobase C, wherein the nucleotide analogue of the fourth type is provided by

(aa) nucleotide analogue molecules of the fourth type comprising the first detectable label,
(bb) nucleotide analogue molecules of the fourth type comprising the second detectable label, and
(cc) nucleotide analogue molecules of the fourth type comprising the third detectable label;

and

b) identifying the nucleotide analogue type incorporated in the sequencing reaction based on the detectable label(s) of the incorporated nucleotide analogue type thereby determining the sequence of the nucleic acid template.

The core advantages have been explained above and are also described in further detail below. Specific embodiments of the present method are described in the following. It is noted that all embodiments described herein specifically apply to and are disclosed for the preferred embodiment, wherein different fluorescent dyes are used to provide the first, second and third detectable label.

As explained herein and demonstrated in the examples, labelling the four nucleotide analogues used for sequencing with the three different detectable labels using the principles of the present invention, wherein the nucleotide analogue of the fourth type is provided as a mixture of nucleotide analogue molecules of the fourth type, wherein within said mixture, nucleotide analogue molecules of the fourth type are labelled with the first, second or third detectable label, has important advantages. This labelling principle allows to select four codewords encoding the four nucleotides to be deciphered, wherein the codewords advantageously may have a Hamming distance of 2 for robust coding and decoding. In the method according to the first aspect, the nucleotide analogues of the first, second and third type may provide upon detection the binary codewords [001] (=first detectable label detected, i.e. first nucleotide type incorporated), [010] (=second detectable label detected, i.e. second nucleotide type incorporated), and [100] (=third detectable label detected, i.e. third nucleotide type incorporated), and the nucleotide analogue of the fourth type may provide upon detection the binary codeword [111] (= all three detectable labels detected, i.e. fourth nucleotide type incorporated). The advantages of this coding scheme are also described in further detail in the examples. It is self-evident that the different codewords [001], [010] and [100] can be freely assigned to the nucleotide analogue types that are labelled with a single detectable label.

The nucleic acid template may be provided by a plurality of identical polynucleotides. The method may comprise performing repeated sequencing cycles comprising steps a) and b), wherein per sequencing cycle, the same nucleotide analogue type is incorporated into the plurality of identical polynucleotides providing the nucleic acid template. The sequencing method is preferably based on the established sequencing by synthesis principle, preferably sequencing by synthesis by cyclic reversible termination. This principle is commonly used in next generation sequencing applications. As is common in such sequencing methods, the method usually comprises performing repeated sequencing cycles comprising steps a) and b). According to one embodiment, y sequencing cycles are performed, wherein y is at least 20, at least 25, at least 50, at least 75, at least 100, at least 125 or at least 150. Furthermore, the number of sequencing cycles performed may be at least 200, at least 250 or at least 300.

According to one embodiment, the nucleotide analogue of the first type is comprised in a first composition (i), the nucleotide analogue of the second type is comprised in a second composition (ii), the nucleotide analogue of the third type is comprised in a third composition, and the nucleotide analogue of the fourth type is comprised in a fourth composition. The compositions (i) to (iv) may be provided in form of a single mixture. Preferably, not more than one detectable label is present per nucleotide analogue molecule. The first, second and third composition comprising the nucleotide analogue of the first, second and third type each comprise a single type of detectable label that is used for determining the sequence. As described herein it is preferred that all nucleotide analogue molecules that are comprised in the first, second, third and fourth composition and which comprise a detectable label are single-labeled nucleotide analogue molecules.

According to one embodiment, all nucleotide analogue molecules of the first, second, third and fourth type are labelled with a detectable label. However, as is known from the prior art sequencing methods, such as in particular next generation sequencing methods, it is also possible to use a mixture of labeled and non-labeled nucleotide analogues in the sequencing reaction (see e.g. GeneReader, QIAGEN). The additional use of non-labelled nucleotide molecules of the first, second, third and/or fourth type is also within the scope of the present disclosure. Therefore, according to one embodiment a portion of the nucleotide analogue molecules of the first, second, third and/or fourth type do not comprise a detectable label and accordingly, are non-labeled nucleotide analogue molecules. Thus, according to one embodiment,

(i) a portion of the nucleotide analogue molecules of the first type do not comprise a detectable label;
(ii) a portion of the nucleotide analogue molecules of the second type do not comprise a detectable label;
(iii) a portion of the nucleotide analogue molecules of the third type do not comprise a detectable label; and/or
(i) a portion of the nucleotide analogue molecules of the fourth type do not comprise a detectable label.

Accordingly, each of the four nucleotide analogue types may be provided as a mixture of labeled and non-labeled nucleotide analogue molecules. As described herein, the use of optically detectable labels, such as the use of three different fluorescent dyes of different colors, is preferred. The three colors provided by the three different detectable labels can be detected e.g. by taking images. According to one embodiment, a three-color system and three-image system with approximately equal ratios of labeled and non-labeled nucleotides for all colors are used such that the resulting intensities recorded by the optical sensors (e.g. cameras) are about the same magnitude. The amounts of non-labeled and labeled nucleotide analogue molecules of each type may be adjusted to the detectable label intensity, wherein the intensity per detected signal is preferably approximately the same for each nucleotide analogue type comprising a detectable label. This ensures a uniform signal for each nucleotide analogue type which is advantageous for the detection step. Accordingly, the amounts of labelled and non-labelled nucleotide analogue molecules for each type may be adjusted to achieve an equal intensity for the different detectable labels. E.g. if a more intense fluorescent dye is the amount of labelled nucleotide analogue molecules can be reduced. According to one embodiment, a uniform signal is attained across all colors.

The reaction kinetics and thus incorporation efficiencies can differ for different types of nucleotide analogues. Furthermore, unlabeled nucleotide analogues may be incorporated more readily than labeled nucleotide analogues. The concentrations of the different types of labeled and unlabeled nucleotide analogues may be adjusted in order to tailor the incorporation efficiencies and thus the signal provided for each detectable label. The concentrations of the four different types of nucleotide analogues may be adjusted to have equal or about equal incorporation efficiencies. Moreover, knowledge of the incorporation efficiencies may also be utilized to achieve approximately equal detectable label intensities between the different optical signals (e.g., detectable emission). The received signals should preferably roughly be balanced. According to one embodiment, approximately the same signal may be achieved for each detectable label (e.g. color) by keeping the percentage of a specific (e.g. first) detectable label constant between the two different nucleotide analogue types that carry this specific detectable label. For example, in order to obtain in a method according to the first aspect about the same optical signal strength for the first detectable label with the first type of nucleotide analogue as with the fourth type of nucleotide analogue, the percentage of the molecules comprising the first detectable label may be kept constant within the different nucleotide analogue types. E.g. 25% of the nucleotide analogue molecules of the first type may carry the first label while 75% of the molecules of the first type are unlabeled and 25% of the nucleotide analogue molecules of the fourth type carry the first label, while 75% of the molecules of the fourth type do not comprise the first label but comprise either the second or third label or are unlabeled. Furthermore, the concentration (e.g. molar concentration) of the different nucleotide analogue types can be adjusted to have approximately equal incorporation efficiencies for these nucleotide analogue types. Similar adjustment can be performed in order to detect about the same optical signal strength for nucleotide analogues of the second and fourth type, as well as nucleotide analogues of the third and fourth type. It is preferred to balance signals of the detectable labels, respectively the received optical signals (e.g. total detected emission). The concentrations of the four different nucleotide analogue types and the labeled molecules can be accordingly chosen.

According to one embodiment, the mixture of non-labeled and labeled nucleotide analogue molecules of the first, second and third type is adjusted to the detectable label intensity, wherein the intensity of total detected signal of each nucleotide analogue type comprising one detectable label (first, second and third type, see above) is around three times less as the intensity of total detected signal of the nucleotide analogue of the fourth type comprising all three detectable labels.

According to one embodiment

> (i) the sum of labeled and non-labeled nucleotide analogue molecules of the first type is 100%, wherein
>
>> (aa) 15% to 50%, preferably 20% to 35%, more preferably 25%, of the nucleotide analogue molecules of the first type comprise the first detectable label, and
>> (bb) 50% to 85%, preferably 65% to 80%, more preferably 75%, of the nucleotide analogue molecules of the first type do not comprise a detectable label;
>
> (ii) the sum of labeled and non-labeled nucleotide analogue molecules of the second type is 100%, wherein (aa) 15% to 50%, preferably 20% to 35%, more preferably 25%, of the nucleotide analogue molecules of the second type comprise the second detectable label, and (bb) 50% to 85%, preferably 65% to 80%, more preferably 75%, of the nucleotide analogue molecules of the second type do not comprise a detectable label;
> (iii) the sum of labeled and non-labeled nucleotide analogue molecules of the third type is 100%, wherein
>
>> (aa) 15% to 50%, preferably 20% to 35%, more preferably 25%, of the nucleotide analogue molecules of the third type comprise a third detectable label, and
>> (bb) 50% to 85%, preferably 65% to 80%, more preferably 75%, of the nucleotide analogue molecules of the third type do not comprise a detectable label;
>
> (iv) the sum of all labeled and non-labeled nucleotide analogues of the fourth type is 100%, wherein the nucleotide analogue molecules of the fourth type comprise 15% to 50%, preferably 20% to 35%, more preferably 25%, non-labeled nucleotide analogue molecules of the fourth type and 50% to 85%, preferably 65% to 80%, more preferably 75% labeled nucleotide analogue molecules of the fourth type.

As described above, the labelled nucleotide analogue molecules of the fourth type comprise (aa) nucleotide analogue molecules of the fourth type comprising the first detectable label, (bb) nucleotide analogue molecules of the fourth type comprising the second detectable label, (cc) nucleotide analogue molecules of the fourth type comprising the third detectable label. According to one embodiment

> (aa) 15% to 35%, preferably 25%, of the nucleotide analogue molecules of the fourth type comprise the first detectable label,
> (bb) 15% to 35%, preferably 25%, of the nucleotide analogue molecules of the fourth type comprise the second detectable label,
> (cc) 15% to 35%, preferably 25%, of the nucleotide analogue molecules of the fourth type comprise the third detectable label, and
> (dd) optionally 15% to 55%, preferably 20% to 35%, more preferably 25%, of the nucleotide analogue molecules of the fourth type do not comprise a detectable label, wherein the sum of all labeled and non-labeled (if present) nucleotide analogue molecules of the fourth type is 100%.

The nucleotide analogue molecules of the fourth type comprising the first, second and third detectable label may be present in an amount so that the same detection intensity is achieved for each detectable label.

According to one embodiment, the nucleotide analogue molecules of the fourth type comprising the first, second or third detectable label are present in an approx. equal amount each, e.g. in a range of 15% to 33.33% each, 20% to 30% each, more preferably 25% each. Any remaining nucleotide analogue molecules of the fourth type can be provided by non-labelled nucleotide analogue molecules. According to one embodiment, the nucleotide analogue of the fourth type comprises 25% nucleotide analogue molecules of the fourth type comprising the first detectable label, 25% nucleotide analogue molecules of the fourth type comprising the second detectable label, 25% nucleotide analogue molecules of the fourth type comprising the third detectable label and 25% non-labelled nucleotide analogue molecules of the fourth type. As discussed above, if three different fluorescent dyes are used to provide the detectable labels, wherein the different fluorescent dyes have different intensities, the amount of nucleotide analogue molecules comprising the different labels can be adjusted so that the intensity provided by the first, second and third fluorescent label is approx. equal for each fluorescent dye. The above indicated % then apply to the intensity for each fluorescent dye.

The identification of the incorporated nucleotide analogue and hence the sequence of the nucleic acid template is based on the use of single-labelled nucleotide analogue molecules. The sequencing reaction does not require the use nucleotide analogue molecules wherein a single nucleotide analogue molecule comprises two or more detectable labels. However, it is nevertheless within the scope of the present invention that the composition(s) comprising the single-labelled nucleotide analogue molecules additionally comprise minor amounts of dual-labelled nucleotide analogue molecules. In embodiments, at least 80% of the nucleotide analogue molecules that comprise a detectable label and are used for determining the sequence of the nucleic acid template comprise at most one detectable label. In embodiments, the amount is at least 85%, at least 90% and preferably at least 95%. Most preferably, 100% of the nucleotide analogue molecules that comprise a detectable label and are used for determining the sequence of the nucleic acid template comprise at most one detectable label and therefore comprise a single detectable label that is used for sequencing. Hence, the most preferred embodiment, each nucleotide analogue molecule that comprises a detectable label and is used for determining the sequence of the nucleic acid template comprises a single detectable label.

Further characteristics of the nucleic acid template, the sequencing reaction, the used nucleotide analogues and the detection step that can be used in conjunction with the method according to the first aspect are discussed in further detail below.

**The method according to the second aspect**

According to a further aspect, the three detectable label based concept of the present invention is implemented by providing three types of nucleotide analogues as mixture comprising two differently labelled nucleotide analogue molecules, while the fourth type of nucleotide analogue is not labelled.

Hence, according to a second aspect, a method for determining the sequence of a nucleic acid template is provided, said method comprising

a) contacting the nucleic acid template in a sequencing reaction with

(i) a nucleotide analogue of a first type, e.g. comprising the nucleobase A, wherein the nucleotide analogue of the first type comprises (aa) nucleotide analogues molecules of the first type comprising a first detectable label, and (bb) nucleotide analogue molecules of the first type comprising a second detectable label;
(ii) a nucleotide analogue of a second type, e.g. comprising the nucleobase T or U, wherein the nucleotide analogue of the second type comprises (aa) nucleotide analogue molecules of the second type comprising the first detectable label, and (bb) nucleotide analogue molecules of the second type comprising a third detectable label;
(iii) a nucleotide analogue of a third type, e.g. comprising the nucleobase G, wherein the nucleotide analogue of the third type comprises (aa) nucleotide analogue molecules of the third type comprising the second detectable label, and (bb) nucleotide analogue molecules of the third type comprising the third detectable label;
(iv) a nucleotide analogue of a fourth type, e.g. comprising the nucleobase C, wherein the nucleotide analogue of the fourth type does not comprise a detectable label; and

b) identifying the nucleotide analogue type incorporated in the sequencing reaction, wherein the incorporation of the first, second and third type of nucleotide analogue is determined based on the detectable labels of the incorporated type of nucleotide analogue and the incorporation of the fourth type is determined based on the absence of a detectable label.

This labelling principle also allows to select four codewords encoding the four nucleotides to be deciphered, wherein the codewords advantageously may have a Hamming distance of 2 for robust coding and decoding. In the method according to the second aspect, the nucleotide analogues of the first, second and third type may provide upon detection the binary codewords [011] (=first and second detectable labels detected, i.e. first nucleotide type incorporated), [101] (=first and third detectable labels detected, i.e. second nucleotide type incorporated), and [110] (=second and third detectable label detected, i.e. third nucleotide type incorporated), and wherein the nucleotide analogue of the fourth type provides upon detection the binary codeword [000] (= no detectable label detected, i.e. fourth nucleotide type incorporated). Again, the different codewords [011], [101] and [110] can be freely assigned to the nucleotide analogue types that comprise two different labels. However, identifying an incorporated nucleotide analogue type based on the use of an unlabelled nucleotide analogue type as it done in the method according to the second aspect, even though allowing to achieve a Hamming distance of 2, is less preferred because identification based on non-labelled nucleotide analogues is more prone to errors. A differentiation between a non-labelled nucleotide and an error in the method (e.g. loss of template) is difficult. Therefore, the method according to the first aspect is more advantageous compared to the method according to the second aspect.

According to one embodiment, (aa) the nucleotide analogue of the first type is comprised in a first composition (i), the nucleotide analogue of the second type is comprised in a second composition (ii), the nucleotide analogue of the third type is comprised in a third composition, and the nucleotide analogue of the fourth type is comprised in a fourth composition, optionally wherein the compositions (i) to (iv) are provided in form of a single mixture.

A portion of the nucleotide analogue molecules of the first, second and/or third type may not comprise a detectable label. Hence, also in conjunction with the method according to the second aspect it is possible to use a mixture of labelled and non-labelled nucleotide analogues. The amounts of labelled and non-labelled nucleotide analogue types may be adjusted to achieve an equal intensity for the different detectable labels.

Further characteristics of the nucleic acid template, the sequencing reaction, the used nucleotide analogues and the detection step that can be used in conjunction with the method according to the second aspect are discussed in further detail below.

**The methods according to the third aspect**

According to a third aspect, a method for sequencing a nucleic acid template comprising four different nucleobases is provided, said method comprising contacting the nucleic acid template in a sequencing reaction with four different nucleotide analogue types that are complementary to the four different nucleobases of the nucleic acid template, wherein the method comprises detecting three different detectable labels that are comprised in the four different nucleotide analogue types, wherein the four different nucleobases are encoded with four 3-bit codewords generated by signals provided by the three different detectable labels that are comprised in the four different nucleotide analogue types and wherein the four 3-bit codewords have a Hamming distance of 2.

In the method according to the third aspect, the nucleic acid template may be contacted in the sequencing reaction with

(i) a nucleotide analogue of a first type, e.g. comprising the nucleobase A, wherein at least a portion or all of the nucleotide analogue molecules of the first type comprise a first detectable label;
(ii) a nucleotide analogue of a second type, e.g. comprising the nucleobase T or U, wherein at least a portion or all of the nucleotide analogue molecules of the second type comprise a second detectable label;
(iii) a nucleotide analogue of a third type, e.g. comprising the nucleobase G, wherein at least a portion or all of the nucleotide analogue molecules of the third type comprise a third detectable label;
(iv) a nucleotide analogue of a fourth type, e.g. comprising the nucleobase C, wherein the nucleotide analogue of the fourth type comprises

(aa) nucleotide analogue molecules of the fourth type comprising the first detectable label,
(bb) nucleotide analogue molecules of the fourth type comprising the second detectable label, and
(cc) nucleotide analogue molecules of the fourth type comprising the third detectable label.

The advantages of using accordingly labelled types of nucleotide analogue types and suitable and preferred embodiments for the first, second, third and fourth nucleotide analogue type were described above in conjunction with the method according to the first aspect to which it is referred and also below. The nucleotide analogues of the first, second and third type provide upon detection the binary codewords [001], [010], and [100], and wherein the nucleotide analogue of the fourth type provides upon detection the binary codeword [111]. Advantageously, these codewords have a Hamming distance of 2.

In the method according to the third aspect, the nucleic acid template may also be contacted in the sequencing reaction with

(i) a nucleotide analogue of a first type, e.g. comprising the nucleobase A, wherein the nucleotide analogue of the first type comprises (aa) nucleotide analogues molecules of the first type comprising a first detectable label, and (bb) nucleotide analogue molecules of the first type comprising a second detectable label;
(ii) a nucleotide analogue of a second type, e.g. comprising the nucleobase T or U, wherein the nucleotide analogue of the second type comprises (aa) nucleotide analogue molecules of the second type comprising the first detectable label, and (bb) nucleotide analogue molecules of the second type comprising a third detectable label;
(iii) a nucleotide analogue of a third type, e.g. comprising the nucleobase G, wherein the nucleotide analogue of the third type comprises (aa) nucleotide analogue molecules of the third type comprising the second detectable label, and (bb) nucleotide analogue molecules of the third type comprising the third detectable label;
(iv) a nucleotide analogue of a fourth type, e.g. comprising the nucleobase C, wherein the nucleotide analogue of the fourth type does not comprise a detectable label.

The advantages of using accordingly labelled types of nucleotide analogue types and suitable and preferred embodiments for the first, second, third and fourth nucleotide analogue type were described above in conjunction with the method according to the second aspect to which it is referred and also below. The nucleotide analogues of the first, second and third type provide upon detection the binary codewords [011], [101], and [110], and wherein the nucleotide analogue of the fourth type provides upon detection the binary codeword [000]. Advantageously, these codewords have a Hamming distance of 2.

Further characteristics of the nucleic acid template, the sequencing reaction, the used nucleotide analogues and the detection step that can be used in conjunction with the methods according to the third aspect are discussed in further detail below.

**Further embodiments and characteristics of the methods according to the present disclosure**

Further general characteristics and embodiments of the methods according to the first, second and third aspect and further sequencing methods described herein are disclosed in the following.

*Nucleic acid template*

Nucleic acid templates for sequencing as well as their preparation are well known in the art and therefore, do not need to be described herein in detail. Common steps for preparing the nucleic acid template for sequencing usually include steps such as nucleic acid isolation from a biological sample, library preparation, target enrichment and clonal amplification. The nucleic acid template may be immobilized to the surface of a solid support such as a substrate or bead. Such steps may be performed in the methods of the present invention in order to prepare the nucleic acid template for sequencing.

According to one embodiment, the nucleic acid template is a DNA template. The DNA template may have been prepared from RNA by reverse transcription. The nucleic acid template may be at least partially double-stranded. According to one embodiment, the nucleic acid template is a self-priming template. The nucleic acid template may comprise multiple copies of the same strand to be sequenced. The nucleic acid template may comprise sequencing adaptors as are commonly used in the art. The nucleic acid template is at least partially single stranded, at least during the sequencing process, to allow the hybridization of the nucleotide analogue to the complementary base. The DNA template may have a length in a range selected from 50 to 1000, 50 to 800 and 50 to 500 nucleotides. Suitable sizes can be obtained by fragmentation. Suitable techniques are well-known in the art. In one embodiment, the nucleic acid template is provided as nanoball, also referred to as rolony (Rolling Circle Amplified Colony). Methods for providing DNA nanoballs are well-known in the art and therefore, do not need to be described in detail (see e.g. Xu, Mingya, "Nex-Generation Sequencing for Biomedical Applications" (2013), Biomedical Sciences ETDs, Paper 152).

As is known in the art, the nucleic acid template to be sequenced may be immobilized to a surface. Immobilization of the nucleic acid template to the surface may be e.g. achieved covalently or by other bonding mechanisms, such as electrostatic or hydrophobic interactions. Details are known in the prior art. The nucleic acid template can be immobilized by well-known substrate functionalization technologies based on inorganic or organic biofunctionalization moieties or agents (e.g. amine and carboxyl functional groups). Suitable chemical functionalities for attachment are known to the person skilled in the art, and include but are not limited to functional groups such as epoxides, aldehydes, activated carboxylates, amines, azides, thiols, amides, hydrazides and isothiocyanates. immobilization may also be based on hybridization.

In one embodiment, said surface is part of a carrier. According to one embodiment, the nucleic acid template is immobilized on a carrier, such as a particle, preferably a magnetic particle. Preferably, multiple copies of the same nucleic acid template are immobilized on the same carrier, i.e. particle. According to one embodiment, the nucleic acid template is provided by a plurality of identical polynucleotides. For massively parallel sequencing applications, the number of identical polynucleotides is in embodiment at least 500, at least 750 or preferably at least 1000. The prior art nucleic acid templates may be sequenced in conjunction with the present invention.

*Sequencing principles and reagents used*

The methods according to the present invention can be used in conjunction with any sequencing method that is based on the use of nucleotide analogues to identify the sequence and thus the consecutive bases of a nucleic acid template based on used detectable labels. Such sequencing methods are known to the skilled person and are also described in the background of the invention (see e.g. Goodwin, 2016, Yohe et al., 2017, and Masoudi-Nejad, 2013). Preferably, the sequencing method is based on sequencing by synthesis, more preferably sequencing by synthesis by cyclic reversible termination. Such methods are discussed in background of the invention and elsewhere herein (see also e.g. Goodwin 2016, Chen, 2013 and Chen, 2014).

Common sequencing by synthesis (SBS) based methods as are in particular used in next generation sequencing methods share important core features that can also be used in the methods according to the present disclosure. A nucleic acid template, usually comprising hundreds to thousands copies of the polynucleotide strand to be sequenced, may be attached to a support, such as e.g. a bead. The nucleic acid template is then contacted with the nucleotide analogue types according to the present invention which comprise three different detectable labels, such as three different fluorescent dyes of different colors (as described above). A complementary nucleotide analogue is hybridized and attached to the growing oligonucleotide chain with a polymerase, such as a DNA polymerase in case of a DNA template. Suitable polymerases are known in the art and are also described e.g. in Chen, 2014. Suitable reaction conditions to enable complementary nucleotide analogue types to be incorporated are also well-known to the skilled person. For instance, a polymerase may be added and optionally a primer, or a self-priming template may be used. The nucleotide analogues used in the present invention preferably comprise a reversible terminating group, which preferably is a 3'OH capping moiety, which prevents the incorporation of a further nucleotide analogue during the same sequencing cycle. Non-incorporated nucleotide analogues may then be removed, e.g. by performing one or more wash steps. The detectable label(s) incorporated during the sequencing cycle (or the absence thereof) can then be detected and the type of nucleotide attached to the oligonucleotide can be identified. The methods of the present invention may furthermore comprise removing the incorporated detectable label(s) and/or capping moiety and the next synthesis cycle may be carried out, in which a new nucleotide analogue is attached to the growing chain.

By carrying out multiple cycles, the sequence of a growing oligonucleotide chain can be determined in a stepwise manner. The method according to the present invention preferably comprises performing repeated sequencing cycles in order to determine the sequence of the nucleic acid template. In one embodiment, y sequencing cycles are performed, wherein preferably y is at least 10. In embodiments, y is selected from 25 to 1000, 50 to 750, 75 to 750, 100 to 500, 100 to 300 and 125 to 250.

The nucleotide analogue may form a phosphodiester bond with the 3'end of a nucleic acid primer that is hybridized to the nucleic acid template or the 3'end of the nucleic acid template if a self-priming nucleic acid template is used. As a result, the nucleotide analogue is incorporated which is complementary to the residue to be identified. This principle is well-known in the art and therefore, requires no detailed description.

Each nucleotide analogue type specifically binds to a unique DNA or RNA nucleotide in the sequencing process. According to one embodiment, each nucleotide analogue type comprises a different nucleobase selected from A, T or U (depending on whether the nucleic acid template is DNA or RNA), G and C. The four nucleotide analogue types used are complementary to the four different nucleobases of the nucleic acid template. As disclosed herein, the skilled person is free to choose which nucleobase to use to provide the first, second, third or fourth nucleotide analogue type as long as all four types are provided and labelled according to the principles of the present invention. According to one embodiment the first nucleotide analogue type is an analogue of A, the second nucleotide analogue type is an analogue of T or U, the third nucleotide analogue type is an analogue of G and the fourth nucleotide analogue type is an analogue of C.

According to one embodiment, the nucleotide analogues used in conjunction with the present invention comprise nucleotides selected from the group consisting of dATP, dTTP, dUTP, dCTP, dGTP and non-natural nucleotide analogs thereof.

The nucleotide analogues used according to the invention, which may be provided in form of a mixture comprising all four nucleotide analogue types required to determine the nucleobases of the nucleic acid template, are contacted with the nucleic acid template during the sequencing reaction. As is known in the art, such nucleotide analogues can be used as building blocks for DNA synthesis. According to a preferred embodiment, a nucleotide analogue used comprises a nitrogenous base (e.g. A, T (or U), G or C), a five-carbon sugar (ribose or deoxyribose) and at least one phosphate group. Moreover, a cleavable linker can be present in the nucleotide analogue which is attached to a detectable label and/or a reversible terminating group, i.e. a capping moiety, which may cap the 3'-OH group. After incorporating the nucleotide analogue type that comprises a nucleobase complementary to the respective nucleobase of the nucleic acid template, a detection step can take place. The four nucleotide analogues types are distinguishable based on the three detectable label(s) used (or the absence of a detectable label as in the method according to the second aspect) as explained above.

According to one embodiment, the nucleotide analogues comprise a cleavable linker to which the detectable label is attached. Moreover, a nucleotide analogue may comprise a 3'OH capping moiety. In general, a single base extension may take place, wherein the incorporation of a nucleotide analogue is terminated reversibly after addition of one matching nucleotide analogue type. Afterward, the cleavable linker may be cleaved and/or the capping moiety may be removed, enabling incorporation of the next nucleotide analogue, which comprises a nucleobase complementary to the sequential/following nucleobase of the nucleic acid template. Cleavable linkers are well-known in the art (see e.g. Goodwin, 2016 and GeneReader platform) and can be used in conjunction with the present invention.

According to one embodiment, the cleavable linker is a photocleavable linker or a chemically cleavable linker. The cleavable linker may also be enzymatically cleavable. Suitable cleavable linkers are well-known in the art (see e.g. the documents discussed in the background and elsewhere herein, in particular Chen 2013 and Chen 2014) and are also described e.g. in WO2005/084367 and WO2017/139415. If the nucleotide analogue comprises a photocleavable linker, the method may comprise photocleaving the linker to release the detectable label from the nucleotide analogue that was incorporated in the sequencing reaction. E.g. UV light may be used to photocleave the linker. If the nucleotide analogue comprises a chemically cleavable linker, the method may comprise adding a cleave reagent that is suitable to cleave the linker and thereby subsequently release the detectable label after incorporation. The cleave reagent may be comprised in a liquid medium. The cleave reagent may be an enzyme if the linker is enzymatically cleavable. In certain embodiments, the chemically cleavable linker is a disulphide linker. The cleave reagent may comprise a reducing agent. Such technologies are e.g. described e.g. in WO2017/139419 and WO2017/139415 to which it is referred.

The capping moiety that is used as reversible terminating group, herein also referred to as "cap", prevents extension with another nucleotide in the same sequencing cycle. After the complementary nucleotide analogue has been incorporated and unincorporated nucleotide analogues were removed, the chemical moiety capping the 3'-OH group can be removed. This restores the free 3'-OH group of the nucleotide which thereby becomes available for incorporating a further nucleotide analogue in the next sequencing cycle. Removal of the capping moiety is performed after binding to the nucleic acid template. The capping moiety may be released (and the free 3'OH group restored) in parallel to releasing the detectable label. However, the capping moiety may also be removed prior to or subsequent to releasing the detectable label. Suitable capping moieties and suitable conditions to remove the capping moiety are well-known in the art (see e.g. Chen 2013, Chen 2014) and therefore, do not need any detailed description.

According to one embodiment, the nucleotide analogue is a 3'O-blocked reversible terminator or a 3'unblocked reversible terminator. Such terminators are well known in the art and are e.g. described in Chen 2013, Chen 2014 and Kim, 2014 "Synthesis of 3'-O-fluorescently mono-modified reversible terminators and their uses in sequencing-by-synthesis"; Bioorganic & Medicinal Chemistry Letters Volume 24, Issue 1, Pages 209-213. According to a preferred embodiment, the nucleotide analogue is a 3'O-blocked reversible terminator which comprises a reversible terminating group a removable moiety capping the 3'-OH group of the nucleotide. Thus, according to a preferred embodiment, the nucleotide analogues comprise a detectable label and a removable moiety capping the 3'-OH group of the nucleotide as reversible terminating group. Such nucleotide analogues are commonly used in conjunction with sequencing methods that are based on sequencing by synthesis by cyclic reversible termination.

According to one embodiment, the removable moiety capping the 3'-OH group is a cleavable moiety capping the 3'-OH group of the nucleotide. Upon cleavage, the 3'OH group can be restored. Details and suitable embodiments are known in the art. According to one embodiment, the detectable label is attached to the nucleobase of the 3'O-blocked reversible terminator (see e.g. Chen 2013, Chen 2014), which can also be used in conjunction with the invention. According to one embodiment, the detectable label is bound to the cleavable capping moiety in the 3'O-blocked reversible terminator. Thus, the nucleotide analogue comprising the detectable label may comprises a cleavable moiety capping the 3'-OH group of the nucleotide and the detectable label is bound to the cleavable capping moiety. In this embodiment, the cleavable capping moiety not only blocks the 3'-OH group to prevent the incorporation of a further base, but additionally functions as the cleavable linker in order to attach the detectable label. After binding to the nucleic acid template, the cleavable capping moiety is cleaved, thereby releasing the cleavable capping moiety to which the detectable label can be attached and regenerating the 3'-OH group for the next sequencing cycle. The detectable label attached to the capping moiety is thereby released into the liquid medium.

### Detectable labels

Suitable detectable labels that can be used in conjunction with the methods of the present invention are well-known in the art. Common labels described in the art include fluorescent, luminescent, light-scattering and colorimetric labels. Detectable labels can rely on optical properties of the detectant, such as absorption, emission and/or refraction, or may be of a non-optical transducer origin, such as magnetic labels. According to a preferred embodiment, the label is an optically detectable label, such as a fluorescent label, preferably a fluorescent dye. It preferably is a color label. The terms, "color", "colour", "colors" or "colours", which may be written in conjunction with the term "label" (e.g. "color label") correspond to a detectable label. Preferably, the color label is a fluorescent dye. Suitable labels and in particular fluorescent labels such as fluorescent dyes are well known in the art and are also described in the documents referred to herein, see also US2014/0242579. As indicated above, it is highly advantageous to only include a single label per labelled nucleotide analogue molecule.

The detectable label is preferably a fluorescent label such as a fluorescent dye. Accordingly, each detectable label can have a predetermined fluorescence excitation and emission spectrum. In an advantageous embodiment, the spectra of the three detectable labels are different from each other, allowing distinguishing the detectable labels, respectively the nucleotide analogue type, by the emitted optical signal or signals. Hence, the fluorescent dye or fluorescent dyes per nucleotide analogue type is accordingly specific for the nucleotide analogue type that they are attached to.

The nature of the fluorophore present in the fluorescent label is generally not limited. It may be any fluorophore compatible with labeling of nucleosides/nucleotides and, depending on the intended use of the modified nucleotides, also with subsequent incorporation of the modified nucleotides into a polynucleotide. Appropriate fluorophores are well known to those skilled in the art and may be obtained from a number of commercial manufacturers, such as Molecular Probes Inc. For example, the three fluorescent dyes may emit green, yellow or red light upon excitation. Moreover, a blue fluorescent dye may be combined with any of the prior dyes. Other non-limiting examples are dansyl-functionalised fluorescent moieties, Cy3 and Cy5 fluorescent labels. Other commercially available fluorescent labels include, but are not limited to, fluorescein, rhodamine (including TMR, texas red and ROX), alexa, bodipy, acridine, coumarin, pyrene, benzanthracene and the cyanins. Moreover, silicone rhodamine dyes may be used. For example, applied fluorophores according to this invention may be Alexa Fluor dyes (Molecular Probes; e.g. Alexa 488) or Atto dyes (Atto-tec, e.g. Atto 532). A suitable combination of three detectable labels that can be used in conjunction with the present invention is provided by Alexa 488, Cy5 and ROX.

According to one embodiment, the detectable label is attached to a phosphate which may correspond to a phosphate of the nucleotide analogue, wherein preferably the detectable label is attached to the end phosphate group. Such phosphate labeling, preferably terminal gamma-phosphate labeling, are known in art. Upon incorporation by the polymerase to the primer terminus, a pyrophosphate (PPi) leaving group comprising the detectable label (e.g. a fluorescent label such as a fluorescent dye). Details are e.g. described in Chen, 2014.

*Detection*

The method according to the present invention comprises detecting the used detectable labels, thereby allowing to determine the sequence of the nucleic acid template. Detection devices suitable for various detectable labels commonly used are well known in the art and therefore, do not need any detailed description. As discussed above, the label is preferably an optically detectable label, preferably a fluorescent label such as a fluorescent dye.

According to one embodiment, an optical detection device is used in order to detect and identify the optically detectable label. The detection device may comprise a sensor. Particularly preferred, the sensor is an optical sensor. According to one embodiment, at least two, preferably three optical signals are detected to identify the incorporated nucleotide analogue respectively the complementary nucleotide of the template in each sequencing cycle. The detectable label may be detected while still being attached to the nucleic acid template. Other options are also feasible. In one embodiment, a laser is used to activate the detectable label, which is detected by the optical sensor. Sequencing-by-synthesis systems may concurrently excite individual detectable labels, i.e. fluorescent detectable labels, by matched excitation wavelengths to the used fluorescent detectable labels and taking an optical signal of all concurrently excited fluorescent detectable labels. A variation could be to concurrently excite all fluorescent detectable labels but to partition the fluorescent responses into subgroups where an optical signal is taken for each subgroup with corresponding filters to only let subgroup fluorescent detectable labels pass to the respective optical signal.

Detection devices for the detection of four different detectable labels (e.g. prior art 4-color based sequencing systems) typically require multiple filter cubes, which oftentimes need to be physically moved/shifted in order to acquire different optical signals (e.g. emission spectra). Such physical movement/shifting operations of the filter cubes may reduce the throughput and thus the sequencing efficiency. Moreover, switchable filter cubes can require complicated mechanics and adjustments. The present invention can advantageously avoid the use of filter cubes, as a dichroic filter can be applied. A dichroic filter can typically realize separation of up to three optical signals when using standard fluorescent dyes and thus can be applied in scope of the invention (but not for various four-detectable label based systems). Using a dichroic filter has various advantages in comparison to filter cubes, including a lower cost, no requirement of complicated mechanics and adjustment, and a fixed position. Furthermore, dichroic filters can easily be applied and/or assembled into existing systems.

In one embodiment, the number concurrently excited wavelengths in subgroups may vary for each detection step, e.g. in the first detection step two-wavelengths may be excited and in the next only one for example. In a further embodiment, the detection device suitable to detect a characteristic property of the detectable label is an optical detection device, preferably a camera, where the optical signal from the label is directed to the sensor by relaying optics such as a lens, or in case of magnification, a microscopic setup, or in case of optical filtering/scanning, a confocal or line-scanning microscopic setup. In another embodiment, a lens-free wide-field optical detection device according to patent US 8,866,063 or similar can be employed.

An optical signal can correspond to a "signal state," meaning a condition in which a specific signal is produced in the detection event. For example, a nucleotide analogue molecule type can be in a signal state if the attached detectable label is optionally activated and produces an optical signal, e.g. a fluorescence label is excited and emits a signal. An optical signal can correspond to a "dark state," meaning a condition in which a specific signal is not produced in the detection event. For example, a nucleotide analogue molecule type can be in a dark state when the nucleotide analogue molecule type lacks a detectable label or the detectable label does not produce a signal, e.g. is not or insufficiently excited at the provided laser wavelength (see e.g. the method according to the second aspect).

In a preferred embodiment, an optical signal may correspond to an excitation/emission and/or absorption spectrum. More preferably, the optical signal may correspond to an excitation/emission and/or absorption signal at a particular wavelength or section of wavelengths of an excitation/emission and/or absorption spectrum. Also the absence of an excitation/emission and/or absorption signal can correspond to an optical signal, as the absence may allow elimination of nucleic analogue type(s) that are not incorporated. In case a nucleotide analogue type does not comprise a detectable label, the absence of an emission and/or absorption signal corresponds to an optical signal by which the nucleotide analogue can be identified (see e.g. the method according to the second aspect). In a preferred embodiment, the three optical signals have a different excitation/emission and/or absorption signal.

In an advantageous embodiment, the optical signal is a fluorescence signal, wherein the detectable label comprises an optically excitable fluorophore. A fluorescence signal may be emitted in case of fluorophore excitation. The fluorophore may be excited by direct illumination. The fluorophore may be excited by evanescent illumination through a (e.g. monochromatic) light source and guided by total internal reflection (TIR). TIR-guided light can also be filtered by wavelength as needed through the sensor setup. In one embodiment, the fluorophore is excited by light. The light may be adjusted to emit at a specific wavelength. Detection of an optical signal can be achieved by a CCD-sensor (charge coupled device) or a CMOS-sensor (complementary metal-oxide-semiconductor).

### Number of taken optical signals

For maximum throughput of a sequencing system the number of optical signals to be taken per sequencing step (e.g. images) should be as small as possible subject to having no impact on the maximal number of sequencing cycles which defines the read length of the sequence. As signal quality deteriorates with increasing sequencing cycles due to phasing (i.e. number of strands that are lagging) and pre-phasing (i.e. number of strands that are leading), it is beneficial to use longer codewords, i.e. more images per cycle, at least with higher sequencing cycle numbers (or in all sequencing cycles).

As is demonstrated in Example 3, the encoding scheme that can be used in the methods of the present invention also allows to have variable length codewords in the sequencing cycles. In early cycle numbers fewer optical signals, e.g. two (i.e. two label based detection), can be taken per sequencing step (e.g. two images), whereas in later sequencing cycles where the signal-to-noise ratio is usually smaller, more optical signals (three) are taken per sequencing cycle.

In contrast to prior art methods, the detectable label coding of this invention allows in embodiments the acquisition of less optical signals than provided detectable labels. In an advantageous embodiment of the present invention, it is sufficient to take two optical signals (e.g. images) to identify the nucleotide analogue type incorporated in a sequencing cycle and thereby the sequence of the nucleic acid template. As is demonstrated in Example 3, already two signals may be sufficient to securely distinguish the nucleotide analogue types, at least in early sequencing cycles. In an exemplary embodiment, an optical signal which can originate from a first detectable label and an optical signal which can originate from a second detectable label can result in following detectable signals: no signal, optical signal from first detectable label, optical signal from second detectable label or optical signals from first and second detectable label. A resulting binary code may be: [00], [01], [10], or [11]. Due to the supplied detectable label coding, these binary codes correspond to portions of the 3-bit encoding scheme that has a Hamming distance of 2, e.g. [001], [010], [100], and [111], respectively (see e.g. method according to the first aspect), or in an alternative embodiment, [000], [011], [101], and [110], respectively (see e.g. method according to the second aspect).

However, in an embodiment of the present invention, acquisition of two optical signals is sufficient to differentiate the detectable labels of nucleotide analogues within the first sequencing cycles, wherein the first sequencing cycles may be from 1 to 50, and in later cycles the signal-to-noise ratio may decrease, necessitating an acquisition of three optical signals. For instance, cycle numbers above 50 may require acquisition of 3 optical signals. Thus, in an embodiment of the methods according to the present disclosure not more than three optical signals, preferably images, are taken per sequencing cycle, optionally wherein in early sequencing cycles 2 optical signals are taken per sequencing cycle and in later sequencing cycles 3 optical signals are taken per sequencing cycle. Early sequencing cycles may be from cycles 1 to 50. Later sequencing cycles may be > 50 sequencing cycles. As disclosed herein detectable labels may be detected by an optical sensor, optionally wherein the optical sensor takes an image. Hence, in one embodiment a sequencing run utilizes a two-channel base calling or a three-channel base calling.

*Coding and decoding*

Coding theory comprises the study of properties of codes and their respective fitness for specific applications, which can inter alia be the transmittal of a message in an effective and accurate manner. An object of coding theory is the enablement of error correcting codes, which is required when interferences (e.g. background noises) exist. In case of sequencing a nucleic acid template, potential interferences comprise residual lead and lag signals within the same detection frame (e.g. same sample emission spectra). In order to allow error detection and correction the resulting code should have an adequate fitness. A measure of the fitness the Hamming distance, which corresponds to the number of positions in which two binary codewords disagree. Accordingly, a Hamming distance h allows detection of h-1 errors and correction of h-2 errors. Therefore, a Hamming distance of 2 allows detection of 1 error, while a Hamming distance of 1 does not allow detection of errors.

The present invention utilizes three different labels for the differentiation of four nucleotide analogue types, wherein each nucleotide analogue type is labeled such that detection corresponds to a 3-bit codeword. These codewords can be [001], [010], [100], and [111] (see method according to the first aspect) or, alternatively, [011], [101], [110], [000] (see method according to the second aspect). As can be derived from the binary code, the Hamming distance is always 2, which allows for robust decoding.

Furthermore, detectable label-mixing (e.g. dyes) and detectable label-coding according to the present invention can be extended to multi-wavelength excitation and corresponding n-ary encoding schemes, where n is larger than two as in the binary encoding, as follows:

The number of optical signals that may be detected corresponds to the length of the coding word and the i-th digit of the coding word has $n_i$ levels corresponding to the number of distinguishable detectable labels in the optical signal(s) plus one (for no colour). Therefore, in a three-detectable label system, a two-subgroup system could have subgroups {0,r,g} and {0,r,b}, respectively, creating codewords {00,0r,0b,r0,rr,rb,g0,gr,gb} if all the colours in a subgroup are distinguishable, for example by an intensity modulation where 'r' has half the intensity of 'g'. Otherwise, the number of distinguishable codewords collapses to the product of distinguishable levels in a subgroup over the number of subgroups. An intensity modulation may be achieved, for example, by either changing labeled/unlabeled nucleotide analogue molecule type concentration ratio, or, wavelength specific transmission ratios of emission filters. As long as there are four unique codewords, decoding is possible. The larger the total number of codewords, the more robust the decoding will be provided that all codewords are equally well detectable. Optimization of such codewords yields then an optimization of the system performance.

**The kit according to the fourth aspect**

According to a fourth aspect, a kit is provided for determining the sequence of a nucleic acid template, wherein the kit comprises
per variant A

(i) a nucleotide analogue of a first type, e.g. comprising the nucleobase A, wherein at least a portion or all of the nucleotide analogue molecules of the first type comprise a first detectable label;
(ii) a nucleotide analogue of a second type, e.g. comprising the nucleobase T or U, wherein at least a portion or all of the nucleotide analogue molecules of the second type comprise a second detectable label;
(iii) a nucleotide analogue of a third type, e.g. comprising the nucleobase G, wherein at least a portion or all of the nucleotide analogue molecules of the third type comprise a third detectable label;
(iv) a nucleotide analogue of a fourth type, e.g. comprising the nucleobase C, wherein the nucleotide analogue of the fourth type comprises

(aa) nucleotide analogue molecules of the fourth type comprising the first detectable label,
(bb) nucleotide analogue molecules of the fourth type comprising the second detectable label, and
(cc) nucleotide analogue molecules of the fourth type comprising the third detectable label; or

per variant B

(i) a nucleotide analogue of a first type, e.g. comprising the nucleobase A, wherein the nucleotide analogue of the first type comprises (aa) nucleotide analogues molecules of the first type comprising a first detectable label, and (bb) nucleotide analogue molecules of the first type comprising a second detectable label;
(ii) a nucleotide analogue of a second type, e.g. comprising the nucleobase T or U, wherein the nucleotide analogue of the second type comprises (aa) nucleotide analogue molecules of the second type comprising the first detectable label, and (bb) nucleotide analogue molecules of the second type comprising a third detectable label;
(iii) a nucleotide analogue of a third type, e.g. comprising the nucleobase G, wherein the nucleotide analogue of the third type comprises (aa) nucleotide analogue molecules of the third type comprising the second detectable label, and (bb) nucleotide analogue molecules of the third type comprising the third detectable label;
(iv) a nucleotide analogue of a fourth type, e.g. comprising the nucleobase C, wherein the nucleotide analogue of the fourth type does not comprise a detectable label.

The kit according to variant A can be advantageously used in the method according to the first aspect. The advantages of using accordingly labelled types of nucleotide analogues and suitable and preferred embodiments for the first, second, third and fourth nucleotide analogue type were described above in conjunction with the method according to the first aspect and the generic description to which it is referred. The same disclosure applies here. The kit according to variant B can be advantageously used in the method according to the second aspect. The advantages of using accordingly labelled types of nucleotide analogues and suitable and preferred embodiments for the first, second, third and fourth nucleotide analogue type that can be used per Variant A and B were described above in conjunction with the method according to the second aspect and the generic description to which it is referred. The same disclosure applies here.

Details regarding suitable and preferred nucleotide analogue types were disclosed above and it is referred to the above disclosure which also applies here. The nucleotide analogues of the first, second, third and fourth type may be comprised in variant A and/or Variant B in a single reagent mixture. The kit may also comprise a mixture of labelled and non-labelled nucleotide analogue molecules. Details were explained above and it is referred to the above disclosure which also applies here.

The kit may additionally comprise further reagents that are used in a sequencing by synthesis reaction. The kit may e.g. comprise one or more of the following:

(a) a polymerase;
(b) one or more primers;
(c) a cleavage reagent for cleaving a cleavable linker comprised in a nucleotide analogue molecule;
(d) one or more wash buffers.

Such common reagents are well-known in the art and were also addressed above.

**Further aspects of the present disclosure**

According to a further aspect, a method for sequencing a nucleic acid template comprising n different nucleobases is provided, the method comprising (a) contacting the nucleic acid template in a sequencing reaction with n different nucleotide analogue types that are complementary to the n different nucleobases of the nucleic acid template, wherein each of the n-1 nucleotide analogue types is differently single-labelled by just one of n-1 different detectable labels and wherein the nucleotide analogue molecules of the $n^{th}$ nucleotide analogue type are single-labelled by each of the n-1 detectable labels and (b) detecting the incorporated nucleotide analogue type, wherein preferably, n is 4. In one embodiment, the sequencing reaction may comprise a mixture of the $1^{st}$ to $(n-1)^{th}$ nucleotide analogue type, each of which comprises 100/n% single-labeled nucleotide analogue molecules and (100-100/n)% of the respective nucleotide analogue molecule which is unlabeled, and further comprising a reagent mixture of the $n^{th}$ nucleotide analogue type which comprises (100/n)% single-labeled nucleotide analogue molecules for each of the n-1 labels and (100/n)% of the nucleotide analogue molecules being unlabeled. n corresponds to the number of different nucleobases comprised in the nucleic acids template (and hence also the number of provided nucleotide analogue molecules) and preferably n=4. The method may have the characteristics as defined in claims 11 to 16. Details regarding the detectable labels, templates, nucleotide analogue types and further characteristics of such sequencing methods are also described above in the further embodiments and characteristics of the methods according to the present disclosure and it is referred to the above disclosure which also applies here.

According to a further aspect, a computer program is provided, wherein the computer program is stored on a computer-readable memory, the computer program for determining the sequence of a polynucleotide comprising 4 different nucleobases, the computer program comprising instructions to identify three different detectable labels and to code the identified detectable labels using a 3-bit code for each nucleobase, wherein the codewords for the 4 different nucleobases have a Hamming distance of 2. The computer program may comprise instructions to code the identified labels based on $2^{(n-1)}$ bits of information, wherein n = 4.

According to a further aspect, the present disclosure pertains to the use of a coding in a determination of a sequence of a nucleic acid template comprising 4 different nucleobases, wherein the 4 nucleobases are encoded by 4 3-bit codewords that have a Hamming distance of 2. Details of the coding and decoding scheme and particular advantages are described elsewhere and it is referred to this disclosure. A coding based on $2^{(n-1)}$ bits of information may be used, wherein n = 4.

This invention is not limited by the exemplary methods, uses, systems and materials disclosed herein, and any methods, uses, systems and materials similar or equivalent to those described herein can be used in the practice or testing of embodiments of this invention. Numeric ranges are inclusive of the numbers defining the range. The headings provided herein are not limitations of the various aspects or embodiments of this invention which can be read by reference to the specification as a whole.

As used in the subject specification, the singular forms "a", "an" and "the" include plural aspects unless the context clearly dictates otherwise. Reference to "the disclosure" and "the invention" and the like includes single or multiple aspects taught herein; and so forth. Aspects taught herein are encompassed by the term "invention".

It is preferred to select and combine preferred embodiments described herein and the specific subject-matter arising from a respective combination of preferred embodiments also belongs to the present disclosure.

**EXAMPLES**

**1. Example 1**

For sequencing, a three detectable label based system as described herein provides an equivalent information content as a 4-detectable label based system and is superior to a two detectable label based system. This will be explained below by comparing systems that use either 2, 3 or 4 different colors for the detectable labels:

- The use of 2 colors provides $2^2$ = 4-bit of information. For sequencing, 4 codewords are required, 1 codeword for each nucleotide type comprised in the nucleic acid template. A 2 color based system thus allows a Hamming distance of 1.

  • The use of 3 colors provides $2^3$ = 8-bit of information. This allows to choose 4 codewords having a Hamming distance of 2 (i.e. at least 2 bit differ among all pair-wise codeword comparisons).
  • The use of 4 colors provides $2^4$ = 16-bit of information. This allows to choose 4 codewords having a Hamming distance of 2.

The possible codewords for multi-color coded systems are illustrated in the subsequent Table 1. 4 possible codewords are highlighted in bold. Preferred codewords for the 3 and 4 color based systems having a Hamming distance of 2 are additionally underlined.

| 2 Colours | 3 Colours | 4 Colours |
|-----------|-----------|-----------|
| **00** | 000 | 0000 |
| **01** | **001** | **0001** |
| **10** | **010** | **0010** |
| **11** | 011 | 0011 |
| | **100** | **0100** |
| | 101 | 0101 |
| | 110 | 0110 |
| | **111** | 0111 |
| | | **1000** |
| | | 1001 |

(continued)

| 2 Colours | 3 Colours | 4 Colours |
|---|---|---|
| | | 1010 |
| | | 1011 |
| | | 1100 |
| | | 1101 |
| | | 1110 |
| | | 1111 |

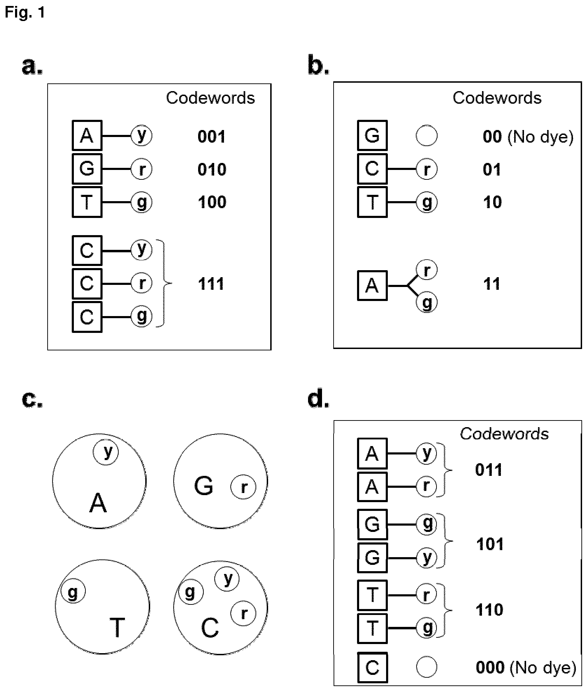

According to the coding theory, a binary code with a Hamming distance n can detect n-1 errors and correct t errors, where t is the largest integer number smaller or equal than (n-1)/2. A 2-detectable label based system therefore does not allow to detect and correct errors (see also **Fig. 1**).

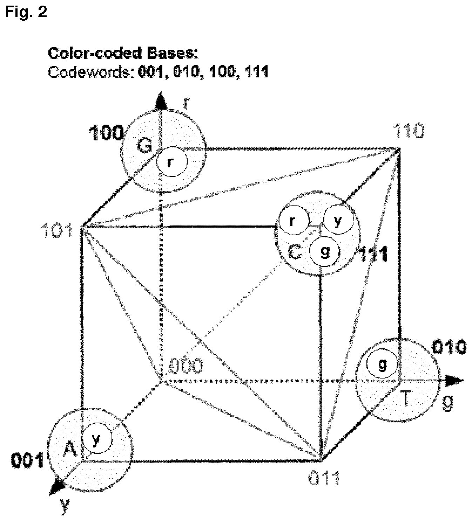

On binary coding level, 3- and 4-detectable label based systems are essentially equivalent. For decoding, the use of analogue signal levels allows to correct errors in the 3- and 4-detectable label systems. With sufficiently accurate lead/lag estimation, the 3-detectable label based system can recover full information of a conventional 4-colour system. Robust decoding is possible with up to 1/3 lead/lag error intensity (can detect for 1 colour error (and also correct because only a subset of codewords is used and one can additionally use the analog level information)). The estimate lead/lag is sufficiently accurate such to correct an error below 1/3 of the normal intensity. This allows robust decoding and an error-free base call in the 3-detectable label based system of the present invention (**Figs. 2 and 3** show the principle of 3-detectable labels coded bases enabling robust decoding). The robust decoding allows up to 1/3 lead/lag still error-free decoding, leading to similar readout quality and read length expected as the current 4-detectable label based system. The advantages of the present invention include a higher throughput (increase by 33% = factor 4/3). Furthermore, 3 signal detection sensors (e.g. cameras) in parallel can be applied or only 1 signal detection sensor (e.g. RGB camera) thereby, allowing to increase the throughput by +400%. E.g., applying 3 cameras in parallel enables a throughput factor of 4. 1 RGB-Camera System enables a throughput factor of 4 without the necessity of registration over the colour channels. Advantageously, the present invention allows to use well-established nucleotide analogues and therefore, can be based on the established sequencing chemistry. It does not require the use of nucleotides that are conjugated to two detectable labels (dual-labelled nucleotides). Dual-labelled nucleotides are harder to be integrated by the polymerase and therefore are disadvantageous.

Advantages of the present invention are also illustrated in **Fig. 1** based on the use of three different fluorescent dyes, here yellow (y), red (r) and green (g) as detectable labels. Shown is a comparison of an embodiment of the three-colour system (**Fig. 1a**) of the present invention with a prior art two-colour system (**Fig. 1b**), such as a sequencing system by Illumina. In the embodiment of the invention (which is e.g. used in the method according to the first aspect), three nucleotide analogue types comprise a single detectable label (y, r or g) and the fourth nucleotide analogue type is provided as mixture that comprises all three detectable labels (y, r and g). All nucleotide analogue molecules comprise only a single detectable label. In the shown example, the nucleotide A is conjugated to a yellow label, the nucleotide G is conjugated to a red label and the nucleotide T is conjugated to a green label. For C, a mixture is provided, wherein the nucleotide C is conjugated to all three labels, wherein, however, each nucleotide molecule is single-labelled with a yellow, red or green label. In the embodiment shown in Fig. 1a, the 4 codewords for the 3 colour based system are: [001], [010], [100], and [111], wherein the Hamming distance is 2. As will be appreciated by the skilled person, different colors and/or assignment of colors to the individual nucleotides may also be used and the codewords [001], [010], [100] may also be assigned to different colors/nucleotides than illustrated in this embodiment (e.g. [001] for G, [010] for T and [100] for A). Advantageous and important is the use the corresponding codewords having a Hamming distance of 2 to encode the four nucleotides comprised in the nucleic acid template to be sequenced. To counter the lower concentration in the fluorescent dies in the fourth nucleotide analogue type, the ratio of dye-labelled to non-labelled nucleotides may be increased by a factor of 3. A reduced signal strength may also be compensated by the choice of the fluorescent label types and/or an increased amount of excitation light.

A suitable embodiment for providing four nucleotide analogue types with three different labels is illustrated in **Fig. 1c**. For the first nucleotide analogue type (here: comprising the nucleotide A), a single-color nucleotide mix is provided which comprises 25% color-labelled nucleotides (here: yellow) and 75% non-labelled nucleotides. For the second nucleotide analogue type (here: comprising the nucleotide T), a single-color nucleotide mix is provided which comprises 25% color-labelled nucleotides (here: green) and 75% non-labelled nucleotides. For the third nucleotide analogue type (here: comprising the nucleotide G), a single-color nucleotide mix is provided which comprises 25% color-labelled nucleotides (here: red) and 75% non-labelled nucleotides. For the fourth nucleotide analogue type (here: comprising the nucleotide C) a tri-color labelled nucleotide mix is provided which comprises 75% tri-color labelled nucleotide analogue molecules (each single-color (y, g, r) at 25%) and 25% non-labelled nucleotides. The use of 75% of tri-colour labelled nucleotide analogue molecules (each single-colour at 25%) for the fourth type allows to compensate a potentially weaker signal. Thereby, all individual detectable labels are present in an equal amount respectively intensity. The different nucleotide analogue types may be provided in form of separate compositions (i) to (iv), or may be combined in a single reagent mixture comprising all four nucleotide analogue types. As explained herein, the nucleic acid template to be sequenced may be immobilized to a solid support, such as a bead, during sequencing. After each sequencing cycle, the bead acquires due to the incorporated nucleotide analogue a specific colour, in the shown embodiment yellow for A, green for T, red for G and yellow, green and red for C. It is preferred to achieve equal bead channel intensities for all 4 nucleotides. This assuming no cross-talk from optics. This can be achieved by optimizing the concentrations of the labelled nucleotide analogue molecules in the reagent mixture and/or chosing fluorescent dyes of appropriate intensities. Furthermore, the optical filters may be adapted to achieve or support that result. Hence, in a preferred embodiment, the signal intensity of the labels is adjusted to have around equal intensity/strength per detection frame or channel, i.e., in case of optical detection, around equal emission signal strength per emission spectrum. Selected concentrations in the reagent mix lead to equal channel intensities for all 4 nucleotides, assuming no cross-talk from the optics, which may be further optimized by adapting the concentrations and/or change optical filters.

In comparison, the 2-colour based system of the prior art comprises of one unlabelled nucleotide analogue type (here: G), two single labelled nucleotide types (C conjugated to a red label and T conjugated to a green label), and one double labelled nucleotide (A, wherein the nucleotide molecules are labelled with red and green). The 4 codewords are: [00], [01], [10], and [11], wherein the Hamming distance is 1 (not allowing error detection and correction by decoding). The dual-dye linked nucleotide analogue is less efficiently integrated by the polymerase during synthesis.

Fig. 1d shows a further embodiment of the present disclosure as it is e.g. used in the method according to the second aspect, wherein for the first, second and third nucleotide analogue type a mixture of single-labelled nucleotide analogue molecules is used that comprise two detectable labels (yellow and red for A; green and yellow for G and red and green for T) and the fourth nucleotide analogue type C comprises no detectable label. The 4 codewords used in this embodiment are [000], [011], [101], and [110] and likewise have a Hamming distance of 2.

Fig. 2 demonstrates that the three-colour system of the present invention (as it is e.g. used in the method according to the first aspect) results in a Hamming distance of 2, as can be derived from the system of coordinates, wherein the preferred 4 codewords are [001], [010], [100], and [111]. In the shown embodiment, the (not used) non-codewords [000], [011], [101], and [110] define maximally distant separating hyperplanes. This allows a robust decoding by the nearest neighbour. Noteworthy, different colors and/or assignment of colors to the individual nucleotide analogue types and/or assignment of colors to the individual nucleotide analogue types may also be used and the codewords [001], [010], [100] may also be assigned to different colors/nucleotide analogue types than illustrated in this embodiment (e.g. complementary to Fig. 1a: [001] for A, [010] for G and [100] for T).

## 2. Example 2

Illustrated is a further example for a sequencing by synthesis based sequencing method, wherein multiple copies of the polynucleotide to be sequenced are immobilized to a bead as solid support. Table 2 summarizes the colors of the three fluorescent detectable labels used, the possible and chosen codewords (chosen codewords are highlighted in bold and underlined), the used reagent mixture comprising the single-color labelled nucleotide analogue molecules and the bead color resulting from the incorporation of the complementary nucleotide analogue in a sequencing cycle. The used reagent mix comprising the different single-colored labelled nucleotide analogue types has optimized concentrations of the labelled nucleotide mixture for a 3-colour system, in the shown embodiment wherein C is the tri-coloured nucleotide analogue type used for sequencing. The percentages exemplify a ratio of labelled nucleotide analogues to total nucleotides for each nucleotide.

*Table 2*

| Colours r g y | Encoded Base | Reagent mix |
|---|---|---|
| | | A: Yellow (25% of yellow assigned to A) |
| | | T: Green (25% of green assigned to T) |
| | | G: Red (25% of red assigned to G) |
| | | C: Red, Green, Yellow (75%) |
| | | **Resulting bead colour** |
| 0 0 0 | - | - |
| **0 0 1** | **A** | Pure Yellow |
| **0 1 0** | **T** | Pure Green |
| 0 1 1 | - | - |
| **1 0 0** | **G** | Pure Red |
| 1 0 1 | - | - |
| 1 1 0 | - | - |
| **1 1 1** | **C** | Tri-Colour Bead (r,g,y) |

That this 3-colour based system of the invention provides equivalent information content as the prior art 4-colour system and likewise enables robust decoding, is further explained in **Fig. 3** which demonstrates that lead and lag operation can be compensated by the present invention. The codewords for the different nucleotide analogue types are as indicated in Table 2, i.e. [001] for A, [010] for T, [100] for G, and [111] for C. The threshold for binarization is: $\frac{1}{2}^+$ (max(signal$_n$, signal$_{n+1}$) - min(signal$_n$, signal$_{n+1}$)): indicated by the three red dotted lines in Fig. 3. While the identification of the tri-colour bead nucleotide (here "C") is very clear in case of a strong signal of all three colours above the threshold, wherein a 1/3 lag operation is negligible, decoding of C can also be achieved when all three colours have an equal signal just around the signal threshold e.g. by averaging the signal level over all channels. In case of a weak signal and a 1/3 lead/lag operation, the decoding can be based on the majority, yielding C. Apart from the tri-colour bead based nucleotide C, the other nucleotides A, T, and G, can be well differentiated from each other, even at a 1/3 lead and/or lag operation mode (Figure 3B).

Fig. 3A: In case of a normal operation by first measuring the signal of n (here A) and then n+1 (here T), there is no lead or lag involved and the single-labelled nucleotides are clearly distinguishable. In case of a lag operation 1/3 $A_n$ at the cycle n+1, a stronger signal of cycle n+1 enables clear identification of nucleobase label. A lead operation 1/3 $T_{n+1}$ at cycle n can be differentiated similar to the prior lag operation by the higher signal intensity thereby allowing a robust decoding. A lead/lag operation 1/3 $A_n$ at the cycle n+1 and 1/3 $T_{n+1}$ at cycle n can still be securely decoded because the chosen codewords have a Hamming distance of 2 as is shown in Fig. 3A. Because the other possible codeword is not used for coding the 4 different nucleotide analogue types in the 4 codewords providing a Hamming distance of 2.

Fig. 3B: In case of a normal operation in first measuring the signal of n (here A) and then n+1 (here C), there is again no lead or lag involved and the single-labelled nucleotide A and the tri-colour coded nucleotide C are clearly distinguishable. In case of a lag operation 1/3 $A_n$ at the cycle n+1, the three strong signals of cycle n+1 enable clear identification of the nucleotide C. The nucleotide A can also be securely identified because the codewords have a Hamming distance of 2 and the other possible codeword is not used for coding the nucleotide analogues. A lead operation 1/3 $T_{n+1}$ at cycle n may occur, which can be differentiated as illustrated in the figure. A weak signal of the n+1 position may also be identified by the average level over all channels, which also may be stated as a majority decoder on the number of x. This allows to securely identify C based on the detection of all three labels. A lead/lag operation 1/3 $A_n$ at the cycle n+1 and 1/3 $T_{n+1}$ at cycle n, still allows to identify the n position by the stronger signal at the threshold level, whereas the n+1 position may be identified by comparing the signal intensities. Both codewords are possible in the latter case, but the majority decoding yields the correct n+1 nucleotide.

## 3. Example 3

The three detection label based sequencing system also allows a 2-color encoding. This can be done at least in early sequencing cycles, thereby increasing the throughput, while maintaining a low error rate. This can be done by omitting one colour channel. In this example, the yellow (y) channel is omitted and the red (r) and green (g) channels are used for detection (highlighted in bold and underlined). This is shown in Table 3.

*Table 3*

| Colours r g y | Encoded base |
|---|---|
| **0 0** 1 | A |
| **0 1** 0 | T |
| **1 0** 0 | G |
| **1 1** 1 | C |

The colour channel y can be disabled in first cycles to increase the throughput. The weaker encoding of symbols is compensated by stronger signals and minor phasing in first cycles, so that the error rate is not increased. As the number sequencing cycles increase (thereby increasing the likelihood of errors), the disabled colour channel is enabled in the later cycles to compensate for weaker signals and stronger phasing by stronger encoding.

For example, one may use a 2-color encoding for cycles 1-50, and a 3-colour encoding for cycles 51-100 (and later cycles if performed). This decreases the required time for imaging of the exemplified 100 cycles:

- Compared to a 3-colour based encoding system wherein all three channels are used for detection in all cycles: $1/2 \times 2/3 + 1/2 \times 1 = 5/6 = $ **0.83** (= +20% throughput).
- Compared to a 4-colour based encoding system wherein four channels are used for detection in all cycles: $1/2 \times 2/4 + 1/2 \times 3/4 = 5/8 = $ **0.63** (= +60% throughput)

The data from the 3-colour encoding can be directly used to test a full or partial 2-colour encoding.

### 2-image system with concurrent 4-colour encoding system

In this example, two images i1 and i2 are taken. Each image receives all the colours and each colour can be distinguished in each image by intensity level modulation (0=0, r=1, g=2, b=3; see Table 4). The Hamming distance is 2 for the used codewords, but smaller level differences exist, which might not be problematic for similar reasons of total codeword distances and sufficiently well de-phased signals. In case the colours are not distinguishable, the set of codewords collapses to {00,01,10,11}. Hamming distance is then 0 with no redundancy.

*Table 4*

| images i1 i2 | Encoded base (e.g.) |
|---|---|
| 0 0 | |
| 0 r | A |
| 0 g | |
| 0 b | |
| r 0 | |
| r r | |
| r g | C |
| r b | |
| g 0 | |
| g r | |
| g g | |
| g b | G |

(continued)

| images<br>i1 i2 | Encoded base (e.g.) |
|---|---|
| b 0 | T |
| b r | |
| b g | |
| b b | |

### 2-image system with concurrent 3-colour encoding system

When using a 2-image system in conjunction with a 3-colour encoding system (Table 5), a code derivation can take place.

*Table 5*

| Colours<br>0 r g b | Encoded base (e.g.) |
|---|---|
| 0 0 0 1 | A |
| 0 0 1 0 | C |
| 0 1 0 0 | G |
| 0 1 1 1 | T |

The use of a 3-colour encoding is augmented by the 0-vector. The 4 bases are encoded with a Hamming distance of 2. Moreover, sub-groups codes can be built by virtually deleting one colour, e.g. 'b', then 'r' (Table 6). This corresponds to having an image taken without detecting the deleted colour. To distinguish colours in a single image, intensity modulation is used by doubling the intensity of the non-deleted colour (indicated by weight 2). This simple intensity modulation allows to distinguish, for instance, temporary code c = 010 from temporary code d=011, as otherwise they would collapse to the same code, as 'r' and 'g' could not be distinguished. Temporary codewords have a minimum pair-wise "modified" (sum of digit differences) Hamming distance of 2, thereby providing robust temporary codewords in each image. The combined codeword from both images with temporary codewords again have a Hamming distance of 2 (Table 7). Thereby, robust sequencing is possible even if using a 2-image system in conjunction with the three detectable label based system.

*Table 6*

| Colours | | | | Temporary code |
|---|---|---|---|---|
| 0 | r | g | b | |
| 0 | 0 | 0 | . | a |
| 0 | 0 | 2 | . | b |
| 0 | 1 | 0 | . | c |
| 0 | 1 | 2 | . | d |
| 0 | . | 0 | 1 | e |
| 0 | . | 2 | 0 | f |
| 0 | . | 0 | 0 | a |
| 0 | . | 2 | 1 | g |

*Table 7*

| images I1 I2 | Encoded base (e.g.) |
|:---:|:---:|
| a e | A |
| b f | C |
| c a | G |
| d g | T |

The considerable advantages of this embodiments lies in that only 2 images I1 and I2 must be taken per cycle. Moreover, a strong robust encoding of the 3-colour encoding scheme is nevertheless obtained which makes this very suitable and advantageous for sequencing applications, in particular next generation sequencing.

## 4. Example 4

### 4.1. State-of-the-art 4-detectable label based system

Multiple current encoding techniques are primarily based on a four colour code, wherein each nucleotide is encoded with exactly one colour. For multi-colour coding following assumptions are taken:

- Worst case at cycle n: neighbouring positions (n+1; lead) and (n-1; lag) position with the <u>same</u> nucleotide, e.g. A.
- Lead/Lag influence only from closest neighbours, without loss of generality.

According to one example, both neighbouring positions correspond to the same nucleotide. In that case, neighbouring nucleotide analogues may provide upon detection the binary codewords [1,0,0,0] and the current nucleotide analogue may provide upon detection the binary codeword [0,1,0,0] (see below). Note, the first column of the 4x3 matrix corresponds to cycle n-1, the middle column to current cycle n and the leading nucleotide codeword, which corresponds to the subsequent cycle n+1, is in last column.

Cycle

n-1, n, n+1

$$v_{perfect} = \begin{bmatrix} 0 \\ 1 \\ 0 \\ 0 \end{bmatrix} = \begin{bmatrix} 1 & 0 & 1 \\ 0 & 1 & 0 \\ 0 & 0 & 0 \\ 0 & 0 & 0 \end{bmatrix} \begin{bmatrix} 0 \\ 1 \\ 0 \end{bmatrix} \begin{matrix} \text{No lag} \\ \text{Current} \\ \text{No lead} \end{matrix}$$

$$v_{lag/lead} = \begin{bmatrix} P_{lag} + P_{lead} \\ (1 - P_{lag} - P_{lead}) \\ 0 \\ 0 \end{bmatrix} = \begin{bmatrix} P_{lag} + P_{lead} \\ (1 - P_{lag} - P_{lead}) \\ 0 \\ 0 \end{bmatrix} = \begin{bmatrix} 1 & 0 & 1 \\ 0 & 1 & 0 \\ 0 & 0 & 0 \\ 0 & 0 & 0 \end{bmatrix} \begin{bmatrix} P_{lag} \\ 1 - P_{lag} - P_{lead} \\ P_{lead} \end{bmatrix}$$

$$v_{lag/lead,decoded} = \begin{bmatrix} P_{lag} + P_{lead} \\ 1 - P_{lag} - P_{lead} \\ 0 \\ 0 \end{bmatrix} \overset{Decoding}{\underset{P_{lag} + P_{lead} < \frac{1}{2}}{=}} \begin{bmatrix} 0 \\ 1 \\ 0 \\ 0 \end{bmatrix} \begin{matrix} \text{Decoding} \\ \text{is still} \\ \text{acceptable} \end{matrix}$$

$$v_{lag/lead,decoded} = \begin{bmatrix} P_{lag} + P_{lead} \\ 1 - P_{lag} - P_{lead} \\ 0 \\ 0 \end{bmatrix} \quad \overset{Decoding}{\underset{P_{lag} < \frac{1}{2}, P_{lead} < \frac{1}{2}}{=}} \quad \begin{bmatrix} x \\ x \\ 0 \\ 0 \end{bmatrix} = \begin{bmatrix} 0 \\ 0 \\ 0 \\ 0 \end{bmatrix}, \begin{bmatrix} 1 \\ 0 \\ 0 \\ 0 \end{bmatrix}, \begin{bmatrix} 0 \\ 1 \\ 0 \\ 0 \end{bmatrix} \text{ or } \begin{bmatrix} 1 \\ 1 \\ 0 \\ 0 \end{bmatrix}$$

Decoding
breaks down.

Hence, if the sum of $P_{lag}$ and $P_{lead}$ factors is less than 0.5, correct decoding can be achieved.

In case of 4-colour coding with different neighbours, the following may apply. Here, in current cycle n, the codeword of the nucleotide to be encoded is [0,1,0,0]. The previous nucleotide is encoded as [1,0,0,0] and the next nucleotide is encoded as [0,1,0,0]. Here, the less stringent property of $P_{lead} < 0.5$ and $P_{lag} < 0.5$ is sufficient for correct decoding.

Cycle

n-1, n, n+1

$$v_{perfect} = \begin{bmatrix} 0 \\ 1 \\ 0 \\ 0 \end{bmatrix} = \begin{bmatrix} 1 & 0 & 0 \\ 0 & 1 & 1 \\ 0 & 0 & 0 \\ 0 & 0 & 0 \end{bmatrix} \begin{bmatrix} 0 \\ 1 \\ 0 \end{bmatrix} \quad \begin{array}{l} \text{No lag} \\ \text{Current} \\ \text{No lead} \end{array}$$

$$v_{lag/lead} = \begin{bmatrix} P_{lag} \\ 1 - P_{lag} \\ 0 \\ 0 \end{bmatrix} = \begin{bmatrix} P_{lag} \\ (1 - P_{lag} - P_{lead}) + P_{lead} \\ 0 \\ 0 \end{bmatrix} = \begin{bmatrix} 1 & 0 & 0 \\ 0 & 1 & 1 \\ 0 & 0 & 0 \\ 0 & 0 & 0 \end{bmatrix} \begin{bmatrix} P_{lag} \\ 1 - P_{lag} - P_{lead} \\ P_{lead} \end{bmatrix}$$

$$v_{lag/lead,decoded} = \begin{bmatrix} P_{lag} \\ 1 - P_{lag} \\ 0 \\ 0 \end{bmatrix} \quad \overset{Decoding}{\underset{P_{lag} + P_{lead} < \frac{1}{2}}{=}} \quad \begin{bmatrix} 0 \\ 1 \\ 0 \\ 0 \end{bmatrix} \quad \begin{array}{l} \text{Decoding} \\ \text{is still} \\ \text{acceptable} \end{array}$$

$$v_{lag/lead,decoded} = \begin{bmatrix} P_{lag} \\ 1 - P_{lag} \\ 0 \\ 0 \end{bmatrix} \quad \overset{Decoding}{\underset{P_{lag} < \frac{1}{2}, P_{lead} < \frac{1}{2}}{=}} \quad \begin{bmatrix} 0 \\ 1 \\ 0 \\ 0 \end{bmatrix} \quad \begin{array}{l} \text{Decoding} \\ \text{is still} \\ \text{acceptable} \end{array}$$

In case of 4-colour coding with different neighbours moreover the following may apply. E.g. one neighbouring nucleotide analogue provides upon detection the binary codeword [1,0,0,0] and the other neighbouring nucleotide analogue provides upon detection the binary codeword [0,0,1,0], while the current nucleotide analogue provides upon detection the binary codeword [0,1,0,0]). See below:

$$v_{perfect} = \begin{bmatrix} 0 \\ 1 \\ 0 \\ 0 \end{bmatrix} = \begin{bmatrix} 1 & 0 & 0 \\ 0 & 1 & 0 \\ 0 & 0 & 1 \\ 0 & 0 & 0 \end{bmatrix} \begin{bmatrix} 0 \\ 1 \\ 0 \end{bmatrix}$$

Cycle n-1, n, n+1

No lag
Current
No lead

$$v_{lag/lead} = \begin{bmatrix} P_{lag} \\ 1 - P_{lag} - P_{lead} \\ P_{lead} \\ 0 \end{bmatrix} = \begin{bmatrix} P_{lag} \\ (1 - P_{lag} - P_{lead}) \\ 0 \\ 0 \end{bmatrix} = \begin{bmatrix} 1 & 0 & 0 \\ 0 & 1 & 0 \\ 0 & 0 & 1 \\ 0 & 0 & 0 \end{bmatrix} \begin{bmatrix} P_{lag} \\ 1 - P_{lag} - P_{lead} \\ P_{lead} \end{bmatrix}$$

$$v_{lag/lead,decoded} = \begin{bmatrix} P_{lag} \\ 1 - P_{lag} - P_{lead} \\ P_{lead} \\ 0 \end{bmatrix} \overset{Decoding}{\underset{P_{lag} + P_{lead} < \frac{1}{2}}{=}} \begin{bmatrix} 0 \\ 1 \\ 0 \\ 0 \end{bmatrix}$$

Decoding is still acceptable

$$v_{lag/lead,decoded} = \begin{bmatrix} P_{lag} \\ 1 - P_{lag} - P_{lead} \\ P_{lead} \\ 0 \end{bmatrix} \overset{Decoding}{\underset{P_{lag} < \frac{1}{2}, P_{lead} < \frac{1}{2}}{=}} \begin{bmatrix} 0 \\ x \\ 0 \\ 0 \end{bmatrix} = \begin{bmatrix} 0 \\ 0 \\ 0 \\ 0 \end{bmatrix} \ or \ \begin{bmatrix} 0 \\ 1 \\ 0 \\ 0 \end{bmatrix}$$

Decoding is still acceptable

At lagging cycle n-1 it is [1,0,0,0], at current cycle n it is [0,1,0,0] and at leading cycle n+1 it is [0,0,1,0] (similar for [0,0,0,1]).

**4.2. 3-detectable label based system according to the present disclosure**

The same assumptions as above apply:

Cycle n-1, n, n+1

$$v_{perfect} = \begin{bmatrix} 1 \\ 1 \\ 1 \end{bmatrix} = \begin{bmatrix} 1 & 1 & 1 \\ 0 & 1 & 0 \\ 0 & 1 & 0 \end{bmatrix} \begin{bmatrix} 0 \\ 1 \\ 0 \end{bmatrix}$$

No lag
Current
No lead

$$v_{lag/lead} = \begin{bmatrix} 1 \\ 1 - P_{lag} - P_{lead} \\ 1 - P_{lag} - P_{lead} \end{bmatrix} = \begin{bmatrix} P_{lag} + (1 - P_{lag} - P_{lead}) + P_{lead} \\ 1 - P_{lag} - P_{lead} \\ 1 - P_{lag} - P_{lead} \end{bmatrix} = \begin{bmatrix} 1 & 1 & 1 \\ 0 & 1 & 0 \\ 0 & 1 & 0 \end{bmatrix} \begin{bmatrix} P_{lag} \\ 1 - P_{lag} - P_{lead} \\ P_{lead} \end{bmatrix}$$

$$v_{lag/lead,decoded} = \begin{bmatrix} 1 \\ 1 - P_{lag} - P_{lead} \\ 1 - P_{lag} - P_{lead} \end{bmatrix} \overset{Decoding}{\underset{P_{lag}+P_{lead}<\frac{1}{2}}{=}} = \begin{bmatrix} 1 \\ 1 \\ 1 \end{bmatrix} \quad \text{Decoding is still acceptable}$$

$$v_{lag/lead,decoded} = \begin{bmatrix} 1 \\ 1 - P_{lag} - P_{lead} \\ 1 - P_{lag} - P_{lead} \end{bmatrix} \overset{Decoding}{\underset{P_{lag}<\frac{1}{2},P_{lead}<\frac{1}{2}}{=}} = \begin{bmatrix} 1 \\ x \\ x \end{bmatrix} = \begin{bmatrix} 1 \\ 0 \\ 0 \end{bmatrix}, \begin{bmatrix} 1 \\ 1 \\ 0 \end{bmatrix}, \begin{bmatrix} 1 \\ 0 \\ 1 \end{bmatrix} \, or \, \begin{bmatrix} 1 \\ 1 \\ 1 \end{bmatrix} \quad \text{Decoding breaks down.}$$

The break-down of the code occurs only in the following cases: if rows [0,1,0] or [1,0,1] appear twice. Noteworthy, rows [1,1,1] or [0,0,0] are beneficial for the lead/lag problem (evaluate to 1 or 0, respectively). In the case of rows [1,1,1], lead and lag cancel out and decoding yields 1. In the case of [0, 0, 0], the decoding is also 0, independent of $P_{lead}$ and $P_{lag}$.

The break-downs of multi-colour coding systems occur in following cases, within the condition that $P_{lag}<1/2, P_{lead}<1/2$.

In the 4-detectable label based system:

- A C A, A G A, A T A
- C A C, C G C, C T C
- G A G, G C G, G T G
- T A T, T C T, T G T

Hence, it occurs in a total of 12 cases out of $4^3$ = 64.

In the 3-detectable label based system:

- A C A, G C G, T C T
- C A C, C G C, C T C
- A G A, A T A
- G A G, G T G
- T A T, T G T

Hence, it likewise occurs in a total of 12 cases out of $4^3$ = 64.

Hence, the same cases potentially fail in both systems. Hence, the 3-detectable label based system is equivalent to the 4-detectable label based system in the sense of being able to handle the lead/lag errors in the same way. Therefore, the 3-detectable label based system has an equivalent content of information as the 4-detectable label based system.

Cited references

Goodwin et al., Nature Reviews, June 2016, Vol. 17: pp. 333 - 351 "Coming of age: ten years of next-generation sequencing technologies"

Yohe et al., Arch Pathol Lab Med, November 2017, Vol. 141: pp. 1544 - 1557 "Review of Clinical Next-Generation Sequencing"

Masoudi-Nejad, Chapter 2 "Emergence of Next-Generation Sequencing in "Next Generation Sequencing and Sequence Assembly" SpringerBriefs in Systems Biology, 2013

Chen et al, 2013 "The History and Advances of Reversible Terminators Used in New Generations of Sequencing Technology" Genomics Proteomics Bioinformatics 11 (2013) 34-40

Chen et al, 2014, "DNA polymerases drive DNA sequencing-by synthesis technologies: both past and present", Frontiers in Microbiology, Volume 5, Article 305, p. 1 to 11

Kim, 2014 "Synthesis of 3'-O-fluorescently mono-modified reversible terminators and their uses in sequencing-by-synthesis"; Bioorganic & Medicinal Chemistry Letters Volume 24, Issue 1, Pages 209-213

Xu, Mingyan, "Next-Generation Sequencing for Biomedical Applications" (2013), Biomedical Sciences ETDs, Paper 152

US2014/0242579; WO2005/084367; WO2017/139415; WO2017/139419

**Claims**

1. A method for determining the sequence of a nucleic acid template comprising

    a) contacting the nucleic acid template in a sequencing reaction with

    (i) a nucleotide analogue of a first type, wherein at least a portion or all of the nucleotide analogue molecules of the first type comprise a first detectable label;
    (ii) a nucleotide analogue of a second type, wherein at least a portion or all of the nucleotide analogue molecules of the second type comprise a second detectable label;
    (iii) a nucleotide analogue of a third type, wherein at least a portion or all of the nucleotide analogue molecules of the third type comprise a third detectable label;
    (iv) a nucleotide analogue of a fourth type, wherein the nucleotide analogue of the fourth type comprises

        (aa) nucleotide analogue molecules of the fourth type comprising the first detectable label,
        (bb) nucleotide analogue molecules of the fourth type comprising the second detectable label, and
        (cc) nucleotide analogue molecules of the fourth type comprising the third detectable label;

    and
    b) identifying the nucleotide analogue type incorporated in the sequencing reaction based on the detectable label(s) of the incorporated nucleotide analogue type thereby determining the sequence of the nucleic acid template.

2. The method according to claim 1, wherein the nucleic acid template is provided by a plurality of identical polynucleotides and wherein the method comprises performing repeated sequencing cycles comprising steps a) and b), wherein per sequencing cycle, the same nucleotide analogue type is incorporated into the plurality of identical polynucleotides providing the nucleic acid template.

3. The method according to claim 1 or 2, wherein the nucleotide analogue of the first type is comprised in a first composition (i), the nucleotide analogue of the second type is comprised in a second composition (ii), the nucleotide analogue of the third type is comprised in a third composition, and the nucleotide analogue of the fourth type is comprised in a fourth composition, optionally wherein the compositions (i) to (iv) are provided in form of a single mixture.

4. The method according to one or more of claims 1 to 3, wherein

    (i) a portion of the nucleotide analogue molecules of the first type do not comprise a detectable label;
    (ii) a portion of the nucleotide analogue molecules of the second type do not comprise a detectable label;
    (iii) a portion of the nucleotide analogue molecules of the third type do not comprise a detectable label; and/or
    (iv) a portion of the nucleotide analogue molecules of the fourth type do not comprise a detectable label.

5. The method according to claim 4, wherein

    (i) the sum of labeled and non-labeled nucleotide analogue molecules of the first type is 100%, wherein

        (aa) 15% to 50%, preferably 20% to 35%, more preferably 25%, of the nucleotide analogue molecules of the first type comprise the first detectable label, and
        (bb) 50% to 85%, preferably 65% to 80%, more preferably 75%, of the nucleotide analogue molecules of the first type do not comprise a detectable label;

    (ii) the sum of labeled and non-labeled nucleotide analogue molecules of the second type is 100%, wherein

        (aa) 15% to 50%, preferably 20% to 35%, more preferably 25%, of the nucleotide analogue molecules of the second type comprise the second detectable label, and
        (bb) 50% to 85%, preferably 65% to 80%, more preferably 75%, of the nucleotide analogue molecules of the second type do not comprise a detectable label;

    (iii) the sum of labeled and non-labeled nucleotide analogue molecules of the third type is 100%, wherein

(aa) 15% to 50%, preferably 20% to 35%, more preferably 25%, of the nucleotide analogue molecules of the third type comprise a third detectable label, and
(bb) 50% to 85%, preferably 65% to 80%, more preferably 75%, of the nucleotide analogue molecules of the third type do not comprise a detectable label;

(iv) the sum of all labeled and non-labeled nucleotide analogues of the fourth type is 100%, wherein the nucleotide analogue molecules of the fourth type comprise 15% to 50%, preferably 20% to 35%, more preferably 25%, non-labeled nucleotide analogue molecules of the fourth type and 50% to 85%, preferably 65% to 80%, more preferably 75% labeled nucleotide analogue molecules of the fourth type, wherein the labelled nucleotide analogue molecules of the fourth type comprise (aa) nucleotide analogue molecules of the fourth type comprising the first detectable label, (bb) nucleotide analogue molecules of the fourth type comprising the second detectable label, (cc) nucleotide analogue molecules of the fourth type comprising the third detectable label.

6.  The method according to claim 5, wherein

(aa) 15% to 35%, preferably 25%, of the nucleotide analogue molecules of the fourth type comprise the first detectable label,
(bb) 15% to 35%, preferably 25%, of the nucleotide analogue molecules of the fourth type comprise the second detectable label,
(cc) 15% to 35%, preferably 25%, of the nucleotide analogue molecules of the fourth type comprise the third detectable label, and
(dd) 15% to 50%, preferably 20% to 35%, more preferably 25%, of the nucleotide analogue molecules of the fourth type do not comprise a detectable label,

wherein the sum of all labeled and non-labeled nucleotide analogue molecules of the fourth type is 100%.

7.  The method according to one or more of claims 1 to 6, wherein the nucleotide analogue molecules of the fourth type comprising the first, second and third detectable label are present in an equal amount.

8.  A method for determining the sequence of a nucleic acid template comprising

a) contacting the nucleic acid template in a sequencing reaction with

(i) a nucleotide analogue of a first type, wherein the nucleotide analogue of the first type comprises (aa) nucleotide analogues molecules of the first type comprising a first detectable label, and (bb) nucleotide analogue molecules of the first type comprising a second detectable label;
(ii) a nucleotide analogue of a second type, wherein the nucleotide analogue of the second type comprises (aa) nucleotide analogue molecules of the second type comprising the first detectable label, and (bb) nucleotide analogue molecules of the second type comprising a third detectable label;
(iii) a nucleotide analogue of a third type, wherein the nucleotide analogue of the third type comprises (aa) nucleotide analogue molecules of the third type comprising the second detectable label, and (bb) nucleotide analogue molecules of the third type comprising the third detectable label;
(iv) a nucleotide analogue of a fourth type, wherein the nucleotide analogue of the fourth type does not comprise a detectable label; and

b) identifying the nucleotide analogue type incorporated in the sequencing reaction, wherein the incorporation of the first, second and third type of nucleotide analogue is determined based on the detectable labels of the incorporated type of nucleotide analogue and the incorporation of the fourth type is determined based on the absence of a detectable label.

9.  The method according to claim 8, wherein the method has one or more of the following characteristics:

(aa) the nucleotide analogue of the first type is comprised in a first composition (i), the nucleotide analogue of the second type is comprised in a second composition (ii), the nucleotide analogue of the third type is comprised in a third composition, and the nucleotide analogue of the fourth type is comprised in a fourth composition, optionally wherein the compositions (i) to (iv) are provided in form of a single mixture;
(bb) a portion of the nucleotide analogue molecules of the first type do not comprise a detectable label;

(ii) a portion of the nucleotide analogue molecules of the second type do not comprise a detectable label; and/or

(iii) a portion of the nucleotide analogue molecules of the third type do not comprise a detectable label.

10. A method for sequencing a nucleic acid template comprising four different nucleobases, comprising contacting the nucleic acid template in a sequencing reaction with four different nucleotide analogue types that are complementary to the four different nucleobases of the nucleic acid template, wherein the method comprises detecting three different detectable labels that are comprised in the four different nucleotide analogue types, wherein the four different nucleobases are encoded with four 3-bit codewords generated by signals provided by the three different detectable labels that are comprised in the four different nucleotide analogue types and wherein the four 3-bit codewords have a Hamming distance of 2.

11. The method according to claim 10, wherein the nucleic acid template is contacted in the sequencing reaction with

(i) a nucleotide analogue of a first type, optionally comprising the nucleobase A, wherein at least a portion or all of the nucleotide analogue molecules of the first type comprise a first detectable label;
(ii) a nucleotide analogue of a second type, optionally comprising the nucleobase T or U, wherein at least a portion or all of the nucleotide analogue molecules of the second type comprise a second detectable label;
(iii) a nucleotide analogue of a third type, optionally comprising the nucleobase G, wherein at least a portion or all of the nucleotide analogue molecules of the third type comprise a third detectable label;
(iv) a nucleotide analogue of a fourth type, optionally comprising the nucleobase C, wherein the nucleotide analogue of the fourth type comprises

(aa) nucleotide analogue molecules of the fourth type comprising the first detectable label,
(bb) nucleotide analogue molecules of the fourth type comprising the second detectable label, and
(cc) nucleotide analogue molecules of the fourth type comprising the third detectable label.

12. The method according to any one of claims 1 to 7 or 10 to 11, wherein the nucleotide analogues of the first, second and third type provide upon detection the binary codewords [001], [010], and [100], and wherein the nucleotide analogue of the fourth type provides upon detection the binary codeword [111].

13. The method according to claim 10, wherein the nucleic acid template is contacted in the sequencing reaction with

(i) a nucleotide analogue of a first type, optionally comprising the nucleobase A, wherein the nucleotide analogue of the first type comprises (aa) nucleotide analogues molecules of the first type comprising a first detectable label, and (bb) nucleotide analogue molecules of the first type comprising a second detectable label;
(ii) a nucleotide analogue of a second type, optionally comprising the nucleobase T or U, wherein the nucleotide analogue of the second type comprises (aa) nucleotide analogue molecules of the second type comprising the first detectable label, and (bb) nucleotide analogue molecules of the second type comprising a third detectable label;
(iii) a nucleotide analogue of a third type, optionally comprising the nucleobase G, wherein the nucleotide analogue of the third type comprises (aa) nucleotide analogue molecules of the third type comprising the second detectable label, and (bb) nucleotide analogue molecules of the third type comprising the third detectable label;
(iv) a nucleotide analogue of a fourth type, optionally comprising the nucleobase C, wherein the nucleotide analogue of the fourth type does not comprise a detectable label.

14. The method according to any one of claims 8, 9 or 13, wherein the nucleotide analogues of the first, second and third type provide upon detection the binary codewords [011], [101], and [110], and wherein the nucleotide analogue of the fourth type provides upon detection the binary codeword [000].

15. The method according to any one of claims 1 to 14, having one or more of the following characteristics:

(i) each nucleotide analogue molecule that comprises a detectable label and is used for determining the sequence of the nucleic acid template comprises a single detectable label;
(ii) the 4 codewords for encoding the different nucleotide analogues having a Hamming distance of 2 are selected from the set of codewords [001], [010], [100], and [111], or the set of codewords [000], [011], [101], and [110];
(iii) at least n-2 detectable labels, preferably n-1 detectable labels, are detected, wherein n corresponds to the number of provided nucleotide analogue types, wherein optionally, the detectable labels are simultaneously detected;

(iv) the detectable label is a optically detectable label, preferably a fluorescent dye;

(v) not more than three optical signals, preferably images, are taken per sequencing cycle, optionally wherein in early sequencing cycles 2 optical signals are taken per sequencing cycle and in later sequencing cycles 3 optical signals are taken per sequencing cycle, optionally wherein early sequencing cycles are from 1 to 50 and later sequencing cycles are > 50 sequencing cycles;

(vi) detectable labels are detected by an optical sensor, optionally wherein the optical sensor takes an image; and/or

(vii) the nucleic acid template has one or more of the following characteristics:

(aa) the nucleic acid template is a DNA template;

(bb) the nucleic acid template is immobilized on a substrate;

(cc) the nucleic acid template comprises multiple copies of the same polynucleotide strand to be sequenced;

(dd) the nucleic acid template is provided as DNA nanoball;

(ee) the nucleic acid template is immobilized on a particle, preferably a magnetic particle, wherein immobilization is optionally achieved by hybridization;

(ff) the nucleic acid template is a self-priming template.

16. The method according to one or more of claims 1 to 15, wherein the nucleotide analogues used have one or more of the following characteristics:

(i) the nucleotide analogue comprises a nucleobase and a reversible terminating group;

(ii) the nucleotide analogue is a 3'O-blocked reversible terminator which comprises as reversible terminating group a removable moiety capping the 3'-OH group of the nucleotide;

(iii) the detectable label is attached to a nucleobase or is bound to a cleavable capping moiety;

(iv) a nucleotide analogue comprising a detectable label comprises a cleavable linker to which the detectable label is attached;

(v) each nucleotide analogue type specifically binds to a unique DNA or RNA nucleotide in the sequencing process;

(vi) the nucleotide analogues comprise nucleotides selected from the group consisting of dATP, dTTP, dUTP, dCTP, dGTP and non-natural nucleotide analogs thereof.

17. A kit for determining the sequence of a nucleic acid template comprising

per variant A

(i) a nucleotide analogue of a first type, wherein at least a portion or all of the nucleotide analogue molecules of the first type comprise a first detectable label;

(ii) a nucleotide analogue of a second type, wherein at least a portion or all of the nucleotide analogue molecules of the second type comprise a second detectable label;

(iii) a nucleotide analogue of a third type, wherein at least a portion or all of the nucleotide analogue molecules of the third type comprise a third detectable label;

(iv) a nucleotide analogue of a fourth type, wherein the nucleotide analogue of the fourth type comprises

(aa) nucleotide analogue molecules of the fourth type comprising the first detectable label,

(bb) nucleotide analogue molecules of the fourth type comprising the second detectable label, and

(cc) nucleotide analogue molecules of the fourth type comprising the third detectable label; or

per variant B

(i) a nucleotide analogue of a first type, wherein the nucleotide analogue of the first type comprises (aa) nucleotide analogues molecules of the first type comprising a first detectable label, and (bb) nucleotide analogue molecules of the first type comprising a second detectable label;

(ii) a nucleotide analogue of a second type, wherein the nucleotide analogue of the second type comprises (aa) nucleotide analogue molecules of the second type comprising the first detectable label, and (bb) nucleotide analogue molecules of the second type comprising a third detectable label;

(iii) a nucleotide analogue of a third type, wherein the nucleotide analogue of the third type comprises (aa) nucleotide analogue molecules of the third type comprising the second detectable label, and (bb) nucleotide analogue molecules of the third type comprising the third detectable label; and/or

(iv) a nucleotide analogue of a fourth type, wherein the nucleotide analogue of the fourth type does not comprise a detectable label.

**Fig. 1**

a.

| Codewords |
|---|
| A — y | 001 |
| G — r | 010 |
| T — g | 100 |
| C — y | |
| C — r | 111 |
| C — g | |

b.

| Codewords |
|---|
| G — ○ | 00 (No dye) |
| C — r | 01 |
| T — g | 10 |
| A — r, g | 11 |

c.

d.

| Codewords |
|---|
| A — y | |
| A — r | 011 |
| G — g | |
| G — y | 101 |
| T — r | |
| T — g | 110 |
| C — ○ | 000 (No dye) |

Fig. 2

Color-coded Bases:
Codewords: 001, 010, 100, 111

**Fig. 3A**

Threshold for binarization: 1/2 * (max(signal$_n$, signal$_{n+1}$) − min(signal$_n$, signal$_{n+1}$) ):

Cycle n: A                                   Cycle n+1: T
    r g y                                        r g y

                    Normal operation

Codeword:   0  0  1                Codeword:   0  1  0
Decoding:      A                   Decoding:      T

                    Lead operation:
                    1/3 A$_0$ at cycle n+1

Codeword:   0  0  1                Codeword:   0  1  0
Decoding:      A                   Decoding:      T

                    Lag operation:
                    1/3 T$_{n+1}$ at cycle n

Codeword:   0  0  1                Codeword:   0  1  0
Decoding:      A                   Decoding:      T

                    Lead/Lag operation:
                    1/3 A$_n$ at cycle n+1
                    1/3 T$_{n+1}$ at cycle n

Codeword:   0  0  1                Codeword:   0  1  0
Readout:    0  x  1  x: 0 or 1     Readout:    0  1  x  x: 0 or 1
As 0 1 1 is not a codeword         As 0 1 1 is not a codeword
→  must be **0 0 1**                →  must be **0 1 0**
**Decoding:      A**                **Decoding:      T**

**Fig. 3B**

Cycle n: A                                    Cycle n+1: C
  r g y                                         r g y
              Normal operation

Codeword:  0 0 1              Codeword:  1 1 1
Decoding:    A               Decoding:    C

                 Lead operation:
                 1/3 A$_n$ at cycle n+1

Codeword:  0 0 1              Codeword:  1 1 1
Readout:   0 0 x,x: 0 or 1   Decoding:    C
As 0 0 0 is not a codeword
→ must be **0 0 1**
**Decoding:    A**

                 Lag operation:
                 1/3 T$_{n+1}$ at cycle n

Codeword:  0 0 1              Codeword:  x x x, x:0 or 1
Decoding:    A               Possible codewords are:
                             000, 001, 100, 111,
                             → Average over all channels
                               (majority decoder on number
                               of x)
                             → Codeword is **1 1 1**
                             **Decoding:    C**

                 Lead/Lag operation:
                 1/3 A$_n$ at cycle n+1
                 1/3 T$_{n+1}$ at cycle n

Codeword:  0 0 x, x:0 or 1   Codeword:  x x 1, x:0 or 1
As 0 0 0 is not a codeword   001 and 111 are both codewords
→ must be **0 0 1**          → Majority decoding yields
**Decoding:    A**                        **1 1 1**
                             **Decoding :    C**

Europäisches
Patentamt

European
Patent Office

Office européen
des brevets

# EUROPEAN SEARCH REPORT

Application Number

EP 19 15 9434

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | SARA GOODWIN ET AL: "Coming of age: ten years of next-generation sequencing technologies", NATURE REVIEWS GENETICS, vol. 17, no. 6, 17 May 2016 (2016-05-17), pages 333-351, XP055544186, GB ISSN: 1471-0056, DOI: 10.1038/nrg.2016.49 * page 340, figure 3 * | 1-17 | INV. C12Q1/6869 G16B30/20 |
| A | WO 2018/129214 A1 (COMPLETE GENOMICS INC [US]; BGI SHENZHEN [CN]) 12 July 2018 (2018-07-12) * page 61 * | 1-17 | |
| A | US 2010/323348 A1 (HAMADY MICAH L [US] ET AL) 23 December 2010 (2010-12-23) * abstract * | 1-17 | |
| A | JP 3 431368 B2 (TOSHIBA CORP) 28 July 2003 (2003-07-28) * abstract * | 1-17 | TECHNICAL FIELDS SEARCHED (IPC) |
| A | US 2013/079232 A1 (BOUTELL J M; ELTOUKHY H A ET AL.) 28 March 2013 (2013-03-28) * claims * | 1-17 | G06F C12Q G16B |

The present search report has been drawn up for all claims

| Place of search | Date of completion of the search | Examiner |
|---|---|---|
| Munich | 26 April 2019 | Hennard, Christophe |

EPO FORM 1503 03.82 (P04C01)

**EP 3 702 474 A1**

## ANNEX TO THE EUROPEAN SEARCH REPORT
## ON EUROPEAN PATENT APPLICATION NO.

EP 19 15 9434

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2019

| Patent document cited in search report | | | Publication date | Patent family member(s) | | | Publication date |
|---|---|---|---|---|---|---|---|
| WO 2018129214 | A1 | | 12-07-2018 | US | 2018223358 | A1 | 09-08-2018 |
| | | | | WO | 2018129214 | A1 | 12-07-2018 |
| US 2010323348 | A1 | | 23-12-2010 | NONE | | | |
| JP 3431368 | B2 | | 28-07-2003 | JP | 3431368 | B2 | 28-07-2003 |
| | | | | JP | H08340258 | A | 24-12-1996 |
| US 2013079232 | A1 | | 28-03-2013 | CA | 2859660 | A1 | 28-03-2013 |
| | | | | DK | 2768972 | T3 | 25-09-2017 |
| | | | | EP | 2768972 | A1 | 27-08-2014 |
| | | | | EP | 3290528 | A1 | 07-03-2018 |
| | | | | ES | 2639938 | T3 | 30-10-2017 |
| | | | | HK | 1201079 | A1 | 21-08-2015 |
| | | | | US | 2013079232 | A1 | 28-03-2013 |
| | | | | US | 2017022559 | A1 | 26-01-2017 |
| | | | | WO | 2013044018 | A1 | 28-03-2013 |

EPO FORM P0459

For more details about this annex : see Official Journal of the European Patent Office, No. 12/82

## REFERENCES CITED IN THE DESCRIPTION

**Patent documents cited in the description**

- WO 2005084367 A
- WO 2017139415 A
- WO 2017139419 A
- US 20140242579 A
- US 8866063 B

**Non-patent literature cited in the description**

- **GOODWIN et al.** Coming of age: ten years of next-generation sequencing technologies. *Nature Reviews,* June 2016, vol. 17, 333-351
- **YOHE et al.** Review of Clinical Next-Generation Sequencing. *Arch Pathol Lab Med,* November 2017, vol. 141, 1544-1557
- Emergence of Next-Generation Sequencing in "Next Generation Sequencing and Sequence Assembly. **MASOUDI-NEJAD.** SpringerBriefs in Systems Biology. 2013
- **CHEN et al.** The History and Advances of Reversible Terminators Used in New Generations of Sequencing Technology. *Genomics Proteomics Bioinformatics,* 2013, vol. 11, 34-40
- **CHEN et al.** DNA polymerases drive DNA sequencing-by synthesis technologies: both past and present. *Frontiers in Microbiology,* 2014, vol. 5, 1-11
- **XU, MINGYA.** Nex-Generation Sequencing for Biomedical Applications. *Biomedical Sciences ETDs,* 2013
- **KIM.** Synthesis of 3'-O-fluorescently mono-modified reversible terminators and their uses in sequencing-by-synthesis. *Bioorganic & Medicinal Chemistry Letters,* 2014, vol. 24 (1), 209-213
- **XU, MINGYAN.** Next-Generation Sequencing for Biomedical Applications. *Biomedical Sciences ETDs, Paper 152,* 2013